(19) Europäisches Patentamt

European Patent Office

Office européen des brevets

(11) **EP 0 714 978 B1**

(12) **FASCICULE DE BREVET EUROPEEN**

(45) Date de publication et mention
de la délivrance du brevet:
**21.03.2001  Bulletin 2001/12**

(51) Int Cl.$^7$: **C11D 17/00**, C09K 7/00,
C10M 115/12, C10L 7/02,
C09K 3/00, C09K 7/08,
C09K 7/02, E21B 33/138

(21) Numéro de dépôt: **95402574.8**

(22) Date de dépôt: **17.11.1995**

(54) **Gel d'un milieu apolaire, son utilisation pour la préparation de fluides de forage à base d'eau**

Gel von nicht-polarem Mitteln, Verwendung bei Herstellung von Bohrspülungen auf Wasser-Basis

Gel of a non-polar medium, and its use for preparing water-based drilling fluids

(84) Etats contractants désignés:
**AT BE CH DE DK ES FR GB GR IE IT LI LU MC NL
PT SE**

(30) Priorité: **28.11.1994  FR 9414213
19.05.1995  FR 9505963
19.05.1995  FR 9505962
17.07.1995  FR 9508604**

(43) Date de publication de la demande:
**05.06.1996  Bulletin 1996/23**

(73) Titulaire: **RHODIA CHIMIE
92408 Courbevoie Cédex (FR)**

(72) Inventeurs:
  • **Aubert, Daniel
    F-94320 Thiais (FR)**
  • **Frouin, Laurent
    F-94240 L'Hay Les Roses (FR)**
  • **Morvan, Mikel
    F-92400 Courbevoie (FR)**

  • **Vincent, Marie-Madeleine
    F-91200 Athis Mons (FR)**

(74) Mandataire: **Fabre, Madeleine-France et al
Rhodia Services,
Direction de la Propriété Industrielle,
40, rue de la Haie-Coq
93306 Aubervilliers Cedex (FR)**

(56) Documents cités:
**EP-A- 0 070 076       EP-A- 0 083 957
EP-A- 0 469 953       WO-A-94/17154
FR-A- 2 516 533       FR-A- 2 690 709
GB-A- 2 084 225       US-A- 4 039 459
US-A- 4 201 678       US-A- 4 422 947
US-A- 5 034 139**

• **SEIFEN,ÖLE,FETTE,WACHSEN, vol. 116, no. 2,
1 Février 1990 AUGSBURG (DE), pages 60-68,
A.BEHLER 'Neue Verdickungsmittel für
Tensideformulierung '**

**Description**

La présente invention a pour objet un gel en milieu apolaire, peu sensible à la température, pouvant être utilisé tel quel en détergence pour le dégraissage des textiles, comme véhicule de matières solides sédimentant en milieu apolaire non gélifié, en cosmétique, ou après dilution en milieu aqueux, comme agent de nettoyage industriel de surface dures, et notamment pour la formulation de fluides forage à base d'eau.

Le document US-A-5.034.139 décrit un gel comprenant un milieu apolaire, un polymère absorbeur d'eau, un agent tensio-actif, un agent à base de phosphore capable de gélifier les hydrocarbures, ledit agent étant le produit de réaction d'un ester phosphate avec un sel d'aluminium basique. Dans ce document, la gélification du milieu apolaire est obtenue uniquement par l'association des sels d'aluminium et des esters de phosphates. Les sels d'aluminium ont pour effet de "réticuler" les esters de phosphate en formant un polymère anionique. Le document EP-A1-0 083 957 décrit des gels organiques mettant également en oeuvre le produit issu de la réaction entre un ester phosphate et un sel alcalin d'aluminium.

Les documents FR-A-2 516 533 et US-A-4.422.947 décrivent l'utilisation d'hydrocolloïdes pour épaissir des milieux aqueux lors de la préparation de fluides de forage.

Selon l'invention, il s'agit d'un gel caractérisé en ce qu'il contient :

- au moins un milieu apolaire (MApo),
- au moins un agent tensio-actif liquide "multicaténaire" sous forme acide (ATMC), soluble ou dispersable dans ledit milieu apolaire,
- au moins un agent de neutralisation (AN) dudit agent tensio-actif "multicaténaire", présent en quantité correspondant à 2 à 30 fois, de préférence 4 à 20 fois la quantité stoechiométriquement nécessaire à la neutralisation dudit agent tensio-actif "multicaténaire" sous forme acide (ATMC),
- de 0,2 à 5, de préférence de 0,3 à 3 molécules d'eau par molécule d'agent tensio-actif liquide "multicaténaire" sous forme acide (ATMC), l'eau étant introduite au moins en partie par l'intermédiaire dudit agent tensio-actif "multicaténaire" sous forme acide (ATMC) et/ou par l'intermédiaire dudit agent de neutralisation (AN),
- éventuellement au moins un agent émulsifiant susceptible d'émulsifier ledit gel dans l'eau ou dans un milieu aqueux,
- et éventuellement au moins une charge solide soluble ou insoluble dans le milieu apolaire (MApo).

On entend par "milieu apolaire" (MApo) tout constituant liquide à la température de préparation dudit gel, qui, situé dans l'espace de solubilité de HANSEN (Handbook of solubility parameters and other cohesion parameters - Allan F.M. BARTON, CRC Press Inc., 1983 -), présente les paramètres suivants :

- $\delta P$ d'interactions Keesom inférieur à 10 $(J/cm^3)^{1/2}$
- $\delta H$ de liaisions hydrogène inférieur à 10 $(J/cm^3)^{1/2}$
- $\delta D$ d'interactions London supérieur à 15 $(J/cm^3)^{1/2}$

A titre d'exemple de milieu apolaire (MApo), on peut citer :

- les triglycérides d'acides gras saturés ou insaturés ayant au moins 12 atomes de carbone et de préférence de 14 à 20 atomes de carbone ; il peut s'agir de triglycérides de synthèse ou de préférence naturels, tels que les huiles végétales du type huile de colza, huile de soja, huile d'arachide, huile de beurre, huile de graine de coton, huile de lin, huile de noix de coco, huile d'olive, huile de palme, huile de pépin de raisin, huile de poisson, huile de ricin, huile de coprah,
- les coupes pétrolières aromatiques,
- les composés terpéniques (D-limonène, L-limonène),
- les mélanges de diesters succinate/adipate/glutarate de diméthyle, dipropyle, diisobutyle, dibutyle,
- les hydrocarbures aliphatiques contenant au moins 6 atomes de carbone (isooctane, kerosène, essence, essence diesel, huiles minérales, huiles lubrifiantes),
- les solvants aromatiques (anisole, toluène),
- les solvants chlorés (trichloro-1,1,1 éthane),
- les huiles essentielles.

On entend par tensio-actif "multicaténaire" (ATMC), tout tensio-actif liquide constitué d'au moins deux chaînes hydrophobes reliées par l'intermédiaire d'une tête hydrophile, ledit tensio-actif étant sous forme acide.

A titre d'exemples, on peut citer les tensio-actifs à base :

---

- de diesters phosphates d'alcools alkoxylés, présentant de 2 à 20, de préférence de 4 à 10 motifs oxyalkylène, de préférence oxyéthylène, et pour lesquels les alcools sont choisis parmi les alcools aliphatiques saturés ou insaturés, linéaires ou ramifiés en $C_6$-$C_{30}$, de préférence en $C_6$-$C_{20}$,
- de diesters phosphates d'alkylphénols alkoxylés, présentant de 2 à 20, de préférence de 4 à 10 motifs oxyalkylène, de préférence oxyéthylène, et pour lesquels les alkylphénols sont choisis parmi ceux dont le radical alkyle est en $C_6$-$C_{30}$, de préférence en $C_6$-$C_{20}$.

Les agents de neutralisation (AN) dudit tensio-actif "multicaténaire" (ATMC), sont de préférence liquides et non solubles dans ledit milieu de suspension apolaire.

Parmi ceux-ci on peut mentionner les amines primaires, secondaires ou tertiaires, les alcanolamines.

A titre d'exemples, on peut citer la triéthanolamine, l'aminométhylpropanol, la cocoamine, la butylamine.

Comme exemple d'agent susceptible d'émulsifier ledit gel de l'invention dans l'eau ou dans un milieu aqueux, on peut citer les tristyrylphénols éthoxylés, les sulfates ou phosphates de tristyrylphénols éthoxylés et/ou propoxylés, les acides ou les alcools gras éthoxylés et/ou propoxylés, les copolymères blocs éthoxylés/propoxylés, le dodécylbenzène sulfonate de sodium.

Les charges solides éventuellement présentes, peuvent être aussi bien des charges inertes que des charges chimiquement actives.

A titre d'exemple, on peut citer notamment :

- des charges du type silice, carbonate de calcium, pigments,
- des hydrocolloïdes susceptibles d'épaissir les milieux aqueux, tels que :

  . les polysaccharides obtenus par fermentation bactérienne (la gomme xanthane, les polysuccinoglycanes, le rhamsan, le welan, le gellan),
  . les galactomannanes (gommes guar et leurs dérivés, caroube, tara),
  . les carraghénanes,
  . les alginates,
  . les dérivés hémisynthétiques de la cellulose tels que les carboxyméthylcelluloses, les méthylcelluloses, les hydroxypropylcelluloses, les hydroxyéthylcelluloses,
  . les polyacrylates de métaux alcalins,
  . les alcools polyvinyliques,
  . les polyéthylène glycols,
  . les polyvinylpyrrolidones,

  seuls ou en association entre eux,
- des agents anti-mousse tels que le stéarate d'aluminium, la silice hydrophobe, l'éthylène bis stéaramide,
- des matières actives comme des composés phytosanitaires,
- des composés basiques solubles dans l'eau tels que métasilicates de sodium, argiles,
- des agents anti-UV tels que l'oxyde de titane, l'oxyde de zinc.

Les caractéristiques viscoélastiques du gel sont fonction à la fois du rapport molaire agent de neutralisation (AN) / tensio-actif "multicaténaire" (ATMC), et des quantités relatives de milieu apolaire (MApo) et de tensio-actif "multicaténaire" (ATMC).

Les quantités de tensio-actif "multicaténaire" (ATMC) sont généralement de 0,5 à 10%, de préférence de 1 à 6% en poids par rapport au poids dudit gel.

Les gels faisant l'objet de l'invention ont l'avantage de présenter des caractéristiques viscoélastiques pouvant aller de celles des gels "souples" (ou "faibles") à celles des gels "durs" (ou "forts").

Un gel est un système viscoélastique, qui peut être caractérisé par un module de conservation G' (traduisant le caractère solide du gel) et un module de perte G" (traduisant le caractère visqueux) en fonction de la vitesse angulaire de déformation.

On entend par gel "dur" (ou "fort") tout gel dont le module de conservation G' présente un plateau dans une gamme de vitesse angulaire allant généralement de $10^{-1}$ à 100 radians/s ; dans cette gamme de vitesse angulaire, G' est au moins 4 fois supérieur à G".

Un gel "dur" (ou "fort") peut être favorablement obtenu à l'aide d'une quantité de tensio-actif "multicaténaire" (ATMC) de 5 à 10 % en poids par rapport au poids dudit gel, avec un rapport molaire agent de neutralisation (AN) / tensio-actif "multicaténaire" (ATMC) de 5 à 10 fois le rapport stoechiométrique de neutralisation, et une quantité d'eau de 0,05 à 0,5% en poids par rapport au poids dudit gel.

On entend par gel "souple" (ou "faible") tout gel dont le module de conservation G' présente un plateau dans une gamme de vitesse angulaire inférieure à 1 radian/s, et reste dans ce plateau supérieur au module de perte G".

Des conditions favorables à l'obtention d'un gel "souple" (ou "faible") correspondent à la présence d'une quantité de tensio-actif "multicaténaire" (ATMC) de 1 à 3% en poids par rapport au poids dudit gel, avec un rapport molaire agent de neutralisation (AN) / tensio-actif "multicaténaire" (ATMC) de 5 à 10 fois le rapport stoechiométrique de neutralisation, et d'une quantité d'eau de 0,01 à 0,1% en poids par rapport au poids dudit gel.

Les gels faisant l'objet de l'invention, peuvent avoir de multiples applications, selon la nature du milieu apolaire (MApo) et la nature des charges éventuelles.

Lesdits gels peuvent être utilisés tels quels, par exemple :

- en détergence, pour le dégraissage des textiles par application dudit gel contenant un solvant actif des graisses comme milieu apolaire, sur la surface à traiter
- comme vehicule de mise en suspension de charges sédimentant naturellement en milieu apolaire non gélifié, notamment comme vehicule d'agents anti-mousse
- en cosmétique pour la préparation de crèmes et gels solaires contenant des agents anti-UV, ou pour la préparation de crèmes de soin dont la phase continue est une huile.

Les gels selon l'invention peuvent également être utilisés après dilution en milieu aqueux comme agents de nettoyage industriel de surfaces dures.

Des gels selon l'invention particulièrement intéressants sont ceux renfermant au moins une charge solide choisie parmi les hydrocolloïdes susceptibles d'épaissir les milieux aqueux, du type de ceux mentionnés ci-dessus.

L'invention concerne donc également un gel (gel G1) spécifique particulièrement utilisable, après dilution dans l'eau, pour la formulation de fluides de forage à base d'eau et comprenant :

- 100 - (a+b+c+d) parties en poids d'au moins un milieu apolaire (MApo),
- une quantité (a) de 0,5 à 6, de préférence de 1 à 4 parties en poids d'au moins un agent tensio-actif liquide "multicaténaire" sous forme acide (ATMC), soluble ou dispersable dans ledit milieu apolaire,
- une quantité (b) d'au moins un agent de neutralisation (AN) dudit agent tensio-actif "multicaténaire", correspondant à 2 à 30 fois, de préférence 4 à 20 fois la quantité stoechiométriquement nécessaire à la neutralisation dudit agent tensio-actif "multicaténaire" sous forme acide (ATMC),
- une quantité (c) d'eau correspondant à 0,2 à 5, de préférence 0,3 à 3 molécules d'eau par molécule d'agent tensio-actif liquide "multicaténaire" sous forme acide (ATMC),
  l'eau étant introduite au moins en partie par l'intermédiaire dudit agent tensio-actif "multicaténaire" sous forme acide (ATMC) et/ou par l'intermédiaire dudit agent de neutralisation (AN),
- et une quantité (d) de 10 à 50, de préférence de 20 à 40 parties en poids d'au moins un hydrocolloïde susceptible d'épaissir les milieux aqueux.

Les hydrocolloïdes susceptibles d'épaissir les milieux aqueux peuvent être choisis parmi la liste définie précédemment et en particulier parmi la gomme xanthane, les polysuccinoglycanes, le rhamsan, le wellan, le gellan.

Selon un mode préféré de l'invention, dans le gel (gel G1), l'hydrocolloïde susceptible d'épaissir les milieux aqueux est un mélange (M) contenant :

- au moins un polysaccharide hydrocolloïde obtenu par fermentation bactérienne (PSB), stable en présence d'ions $Ca^{2+}$ à un pH supérieur à 10, et présentant un indice de pseudoplasticité inférieur ou égal à 0,5 à une concentration de 0,1% en poids dans l'eau distillée
- et au moins un polymère hydrocolloïde d'origine naturelle (PN), stable en présence de ciment et d'ions $Ca^{2+}$ à un pH supérieur à 10, et présentant un indice de pseudoplasticité supérieur ou égal à 0,6 à une concentration de 0,3% en poids dans l'eau distillée
  selon un rapport pondéral PN / PSB de l'ordre de 20/80 à 95/5, de préférence de 50/50 à 90/10.

Le terme "stable" en présence de ciment ou d'ions $Ca^{2+}$ à un pH supérieur à 10, signifie que la viscosité d'une solution aqueuse de l'hydrocolloïde n'est pas affectée par la présence de ciment ou par la présence d'ions $Ca^{2+}$ à un pH supérieur à 10. Par exemple, on peut considérer comme stable, un hydrocolloïde, qui, en solution aqueuse à 0,3% en poids, présente une viscosité, à un gradient de vitesse donné, ne variant pas de plus de $\pm20\%$, de préférence pas de plus de $\pm10\%$ de sa valeur initiale, lorsqu'on lui ajoute 1% en poids par rapport au poids de ladite solution, d'un ciment de type Portland CPA 55® (commercialisé par Les Ciments Français).

L'indice de pseudoplasticité mentionné est celui calculé à l'aide du modèle d'OSTWALD, appliqué à la courbe de rhéologie en écoulement obtenue à l'aide d'un appareil LOW SHEAR commercialisé par CONTRAVES.

Cet indice est déduit de l'équation suivante :

$$\text{Log } \eta = \text{Log K} + (n-1) \text{ Log } \gamma$$

où $\eta$ représente la viscosité en mPa.s , K l'indice de consistance, n l'indice de pseudoplasticité et $\gamma$ le gradient de cisaillement en $s^{-1}$.

Parmi les polysaccharides hydrocolloïdes obtenus par fermentation bactérienne (PSB), pouvant être présents dans le mélange (M), on peut mentionner :

- les succinoglycanes dont le motif de base contient du glucose, du galactose et un reste succinyle ; ils sont décrits dans les demandes de brevet européen EP-A-351 303 et 40 445, ainsi que dans Carbohydrate Research, 73 (1979) pp.159-168, de Clarence A. Knutson ; ils peuvent être obtenus par fermentation microbienne d'un milieu comportant une source de carbone, au moyen d'un microorganisme appartenant au genre *Arthrobacter,* tel que *Arthrobacter stabilis,* en particulier la souche *Arthrobacter stabilis* NRRL-B-1973, au genre *Agrobacterium,* tels *Agrobacterium tumefaciens, Agrobacterium radiobacter* ou, *Agrobacterium rhizogenes,* au genre *Rhizobium,* en particulier *Rhizobium meliloti* et *Rhizobium trifoli,* au genre *Alcaligenes* tel *Alcaligenes faecalis,* en particulier la variété myxogenes ou au genre *Pseudomonas,* en particulier les souches *Pseudomonas sp.* NCIB 11264 et NCIB 11592 ; parmi ces succinoglycanes, on peut tout particulièrement citer les gommes rhéozan, décrites dans la demande de brevet européen EP-A-351 303, et obtenues par fermentation d'une source carbonée au moyen de la souche *Agrobacterium tumefaciens* I-736 déposée à la Collection Nationale de Culture des Microorganismes (CNCM) ;
- les gommes rhamsan dont le motif de base contient du glucose et du rhamnose ; elles peuvent être obtenues par fermentation microbienne d'un milieu comportant une source de carbone, au moyen d'un microorganisme appartenant au genre *Alcaligenes,* de préférence la souche *Alcaligenes* ATCC 31961 ou au genre *Pseudomonas,* en particulier les souches *Pseudomonas paucimobilis,* plus préférentiellement les souches 1-886 déposée à la CNCM et la souche DSM 4429 ; ces polysaccharides sont décrits dans les demandes de brevet européen EP-A-77 680 et 339 445 ;
- les gommes welan dont le motif de base contient du glucose, de l'acide glucuronique, du rhamnose et du mannose ; elles sont décrites dans Jansson PE, Lindberg B et Wildmalm G (1985) Carbohydrate Research 139, 217-223 ; elles peuvent être obtenues par fermentation microbienne d'un milieu comportant une source de carbone, au moyen d'un microorganisme appartenant au genre *Alcaligenes,* de préférence la souche *Alcaligenes* ATCC 31555.

Parmi les polymères hydrocolloïdes d'origine naturelle (PN), on peut mentionner :

- les dérivés hémisynthétiques de la cellulose comme les hydroxyméthylcelluloses, hydroxyéthylcelluloses, hydroxyméthylpropylcelluloses, hydroxypropylcelluloses, carboxyméthylcelluloses ;
- les dérivés alcoxylés de la gomme guar (appelés hydroxyalkylguars), contenant par molécule de 0,01 à 5, de préférence de 0,05 à 0,5 motif(s) oxyalkylène, de préférence oxyéthylène et/ou oxypropylène ; ils peuvent être obtenus par réaction de guar naturelle avec un oxyde d'alkylène (oxyde d'éthylène et/ou de propylène).

Le gel (gel G1) selon l'invention peut comprendre tout particulièrement comme hydrocolloïde un mélange (M) contenant :

- un polysaccharide succinoglycane,
- et un hydroxyalkylguar contenant de 0,01 à 5, de préférence de 0,05 à 0,5 motif(s) oxyéthylène et / ou oxypropylène,

selon un rapport pondéral hydroxyalkylguar / succinoglycane de 20/80 à 95/5, de préférence de 50/50 à 90/10.

Le mélange (M) peut être préparé par mélange à sec de poudres de polysaccharide hydrocolloïde obtenu par fermentation bactérienne (PSB) et de polymère hydrocolloïde d'origine naturelle (PN), par toute méthode connue de l'homme de l'art.

L'invention concerne aussi le procédé de préparation du gel selon l'invention qui consiste à mélanger ses différents constituants, l'agent de neutralisation (AN) et le tensio-actif "multicaténaire" (ATMC) étant introduits chacun séparément dans le milieu apolaire (MApo), l'agent de neutralisation (AN) étant de préférence introduit après le tensio-actif "multicaténaire" (ATMC).

Un mode préférentiel de réalisation consiste à introduire sous agitation mécanique le tensio-actif "multicaténaire" (ATMC) dans le milieu apolaire (MApo) porté à une température de 15 à 50°C, généralement de 25 à 35°C, puis l'agent émulsifiant éventuel, l'agent de neutralisation (AN) et les charges éventuelles.

Lorsque le gel présente la composition du gel G1, il peut être préparé en introduisant sous agitation mécanique dans le milieu apolaire (MApo), l'agent tensio-actif "multicaténaire" (ATMC), puis l'agent de neutralisation (AN), et enfin l'hydrocolloïde.

La présente invention a également pour objet un procédé pour gélifier un milieu apolaire (MApo), ledit procédé étant caractérisé en ce qu'on introduit dans ledit milieu apolaire :

- au moins un agent tensio-actif liquide "multicaténaire" sous forme acide (ATMC), soluble ou dispersable dans ledit milieu apolaire
- au moins un agent de neutralisation (AN) dudit agent tensio-actif "multicaténaire", présent en quantité correspondant à 2 à 30 fois, de préférence 4 à 20 fois la quantité stoechiométriquement nécessaire à la neutralisation dudit agent tensio-actif "multicaténaire" sous forme acide (ATMC)
- de 0,2 à 5, de préférence de 0,3 à 3 molécules d'eau par molécule d'agent tensio-actif liquide "multicaténaire" sous forme acide (ATMC), l'eau étant introduite au moins en partie par l'intermédiaire dudit agent tensio-actif "multicaténaire" sous forme acide (ATMC) et/ou par l'intermédiaire dudit agent de neutralisation (AN)
- éventuellement au moins un agent émulsifiant susceptible d'émulsifier le gel obtenu dans l'eau ou dans un milieu aqueux
- et éventuellement au moins une charge solide soluble ou insoluble dans le milieu apolaire (MApo).

La nature et les quantités respectives des différents constituants en fonction de l'utilisation désirée du gel à obtenir, ainsi que les conditions opératoires ont déjà été mentionnées ci-dessus.

L'invention concerne en outre l'utilisation du gel (gel G1) pour la préparation d'un fluide de forage à base d'eau et en particulier pour la préparation d'un fluide de forage à base d'eau pour la fabrication de parois moulées.

Le procédé de préparation de tels fluides de forage consiste à diluer dans l'eau le gel (gel G1).

En général, pour ce procédé de préparation, on dilue des quantités de gel et d'eau de telle manière que le fluide de forage final comprenne 0,01 à 3 partie(s) en poids d'hydrocolloïde susceptible d'épaissir les milieux aqueux, introduit par l'intermédiaire du gel, pour 100 parties d'eau. De préférence, le fluide de forage comprend de 0,05 à 1,5 partie en poids d'hydrocolloïde pour 100 parties d'eau.

L'utilisation du gel (gel G1) selon l'invention pour la préparation de fluides de forage à base d'eau présente plusieurs avantages.

Tout d'abord, les fluides de forage obtenus ne comprennent que de l'eau et de faibles quantités du gel (gel G1). Le fait que de faibles quantités du gel (gel G1) soient utilisées permet, après utilisation, de mettre le fluide de forage comportant les déblais de terrain en décharge non classifiée. En effet, le gel (gel G1) étant rapidement biodégradable, le stockage des déblais ne nécessitent aucune condition particulière.

Ensuite, les fluides de forage obtenus présentent une rétention d'eau importante, ce qui évite les infiltrations d'eau dans le terrain au cours du percement d'une tranchée, notamment dans le cas de la construction de parois moulées. Les fluides de forage utilisés présentent, en général, un filtrat à 30 min ($V_{30}$) d'au plus 30 ml, de préférence d'au plus 25 ml, encore plus préférentiellement d'au plus 20 ml, la méthode de mesure du filtrat étant celle donnée dans les exemples.

Les fluides obtenus présentent également des propriétés rhéologiques adaptées à leur utilisation pour la fabrication de parois moulées. Ils ont en particulier un comportement pseudo-plastique tel qu'il est possible de les pomper facilement sans qu'il soit nécessaire de mettre en oeuvre des moyens d'agitation très cisaillants.

En général, les fluides de forage obtenus à partir du gel (gel G1) selon l'invention, présentent une viscosité plastique FANN ($V_p$) comprise entre 5 et 15 mPa.s, de préférence comprise entre 5 et 10 mPa.s. Ils présentent également une viscosité MARSH ($V_M$) comprise entre 30 et 140 s, de préférence comprise entre 30 et 100, encore plus préférentiellement comprise entre 35 et 50 s. Ils présentent aussi une yield value (YV) comprise entre 2 et 10 Pa. Ces différentes viscosités sont mesurées selon les tests décrits dans les exemples.

Enfin, les fluides de forage obtenus à partir du gel (gel G1) conservent leurs propriétés après mise en contact avec les déblais du terrain et les ions $Ca^{2+}$ du béton. Ainsi, après séparation des déblais du fluide de forage, il est possible de recycler une importante partie de ce fluide. Ceci est un avantage important par rapport aux fluides connus de l'art antérieur, en général à base de bentonite qui ne sont pas recyclables : ils perdent en effet leurs propriétés rhéologiques après mise en contact avec les ions solubles pouvant être présents dans le terrain ou avec les ions $Ca^{2+}$ du béton.

L'invention concerne enfin l'utilisation du gel (gel G1) en association avec un agent tensio-actif (TA) comme système précurseur d'un fluide de forage sous forme de mousse, pour le percement de tunnels à l'aide d'un tunnelier.

L'invention concerne donc également un système précurseur d'un fluide de forage sous forme de mousse comprenant le gel (gel G1) selon l'invention et un agent tensio-actif (TA).

L'agent tensio-actif (TA) peut être choisi parmi tout type d'agent tensio-actif soluble dans l'eau et compatible avec les hydrocolloïdes susceptibles d'épaissir les milieux aqueux faisant partie du gel (gel G1). Ces agents tensio-actifs doivent permettre de former une mousse et être chimiquement et thermiquement stables dans les conditions de stockage. Il peut s'agir d'un agent tensio-actif cationique, anionique, ionique ou amphotère.

L'agent tensio-actif (TA) du système précurseur de mousse peut en particulier être choisi parmi :

- les alkylsulfates de formule $ROSO_3M$, où R représente un radical alkyle ou hydroxyalkyle en $C_5$-$C_{24}$, de préférence en $C_{10}$-$C_{18}$, M représentant un atome d'hydrogène ou un cation alcalin (sodium, potassium, lithium), ammonium substitué ou non substitué (méthyl-, diméthyl-, triméthyl-, tetraméthylammonium, diméthylpiperidinium) ou dérivé d'une alcanolamine (monoéthanolamine, diéthanolamine, triéthanolamine, monoisopropanolamine) ;
- les bétaïnes, les alkylbétaïnes, les alkyldiméthylbétaïnes, les alkylamidobétaïnes, les alkyltriméthylsulfobétaïnes ;
- les mélanges d'alkylamidobétaïnes et d'alkylsulfosuccinates de sodium où le radical alkyle est en $C_4$ - $C_{18}$ ;
- les alkyléthersulfates $RO(CH_2CHR_1O)_nSO_3M$ où n est compris entre 0,5 et 30 , de préférence entre 0,5 et 10, et où $R_1$ est un groupement alkyl en $C_4$-$C_{18}$, de préférence en $C_{12}$-$C_{15}$ et présentant en moyenne de 0,5 à 30 motifs, de préférence de 0,5 à 10 motifs oxyéthylène et/ou oxypropylène, M représentant un atome d'hydrogène ou un cation alcalin (sodium, potassium, lithium), ammonium substitué ou non substitué (méthyl-, diméthyl-, triméthyl-, tetraméthylammonium, diméthylpiperidinium) ou dérivé d'une alcanolamine (monoéthanolamine, diéthanolamine, triéthanolamine, monoisopropanolamine) ;
- les alpha oléfines sulfonates ;
- les alkylpolyglycosides de formule $R(OCH_2CH_2)_n(G)_m$, où R est un radical hydrocarboné, linéaire ou ramifié, saturé ou insaturé en $C_8$-$C_{24}$, G est un sucre réducteur en $C_5$-$C_6$ tels le galactose, le glucose ou le fructose, n étant compris entre 0 et 20 et m entre 1 et 10 ;
- les alkylamphomonoacétates, les alkylamphodiacétates tels que notamment le cocoamphoacétate de sodium commercialisé sous les dénominations MIRAPON EXCEL® et MIRANOL ULTRA® par RHÔNE-POULENC.

Les quantités respectives en agent tensio-actif (TA) et en gel du système précurseur de fluide de forage sous forme de mousse peuvent être très variables en fonction de l'application du système précurseur. La teneur en tensio-actif (TA) peut ainsi être comprise entre 1 et 2000 % en poids par rapport au poids du gel (gel G1).

Le système précurseur de fluide de forage sous forme de mousse selon l'invention peut comprendre en outre au moins une charge solide autre que les hydrocolloïdes susceptibles d'épaissir les milieux aqueux telle que :

- la silice naturelle (sable, diatomées) ou synthétique (de précipitation),
- le carbonate de calcium, le carbonate de magnésium,
- les oxydes métalliques tels que l'oxyde de fer (hématite, magnétite),
- les pigment naturels (ocre) ou synthétiques,
- des agents régulateurs de mousse tels que le stéarate d'aluminium, la silice hydrophobe, l'éthylène bis stéaramide,
- la chaux naturelle, éteinte, hydroïque,
- le gypse (anhydrite, semihydrate),
- des matières actives comme des composés phytosanitaires,
- des composés basiques solubles dans l'eau tels que métasilicates de sodium, polysilicates, argiles (kaolin, smectite, illite), talcs, mica.

Ces charges solides peuvent représenter au plus 50 % en poids du système précurseur de fluide de forage sous forme de mousse, de préférence au plus 20 %, encore plus préférentiellement au plus 15 %.

La taille des particules est en général comprise entre 0,1 μm et 1 mm, de préférence entre 0,2 μm et 200 μm.

Ces charges peuvent être introduites pour tout ou partie dans le système précurseur par le biais du gel (gel G1). En effet, le gel entrant dans la composition du précurseur peut comprendre des charges solides.

D'une manière générale, lorqu'un faible taux de charges solides est introduit dans le système précurseur, lesdites charges sont totalement introduites par le biais du gel (gel G1).

Les charges solides peuvent également être introduites séparément du gel (gel G1).

L'invention concerne aussi un procédé de préparation de fluide de forage sous forme de mousse qui consiste à diluer dans l'eau le système précurseur défini ci-dessus et à agiter. Cette agitation est générée classiquement par introduction d'un gaz comprimé tel l'air ou l'azote dans le système précurseur de fluide de forage sous forme de mousse dilué dans l'eau.

Le système précurseur est dilué dans l'eau de telle manière que la quantité finale dans l'eau de l'agent tensio-actif (TA) du système précurseur soit en général comprise entre 0,05 et 25 % en poids par rapport à l'eau, de préférence entre 0,05 et 15 %, encore plus préférentiellement entre 0,2 et 1,5 %.

De préférence, la mousse est créée de telle sorte que le coefficient d'expansion K, mesuré à la pression atmosphérique, soit compris entre 5 et 10, le coefficient K étant déterminé par la relation suivante :

$$K = \frac{Vm}{Va}$$

où Vm est le volume de la mousse après activation du système précurseur en solution, et Va est le volume du système précurseur en solution avant activation.

La mousse peut être créée de façon classique dans un générateur de mousse. Par exemple, lors de l'utilisation de la mousse pour le percement de tunnels à l'aide d'un tunnelier, cette mousse peut être injectée par des moyens adaptés, à l'avant du disque de coupe et / ou dans la chambre de confinement où elle se mélange avec les déblais.

L'invention concerne enfin l'utilisation du fluide de forage sous forme de mousse obtenu à l'aide du système précurseur pour l'excavation de tunnels, par exemple à l'aide d'un tunnelier, notamment au moyen d'un bouclier de creusement, tel qu'un bouclier à pression de terre.

La composition du système précurseur de fluide de forage sous forme de mousse (en particulier les quantités respectives de l'agent tensio-actif (TA) et du gel) varie en fonction de la nature du terrain dans lequel le tunnel est excavé. L'homme du métier sait adapter les quantités d'agent tensio-actif (TA) et de gel en fonction du terrain à creuser.

Le fluide de forage sous forme de mousse obtenu à l'aide du système précurseur présente plusieurs avantages dans cette utilisation.

Il permet de diminuer de façon importante les frottements du disque de coupe sur le front de taille.

Il présente une bonne stabilité bien qu'il soit soumis à des pressions supérieures à la pression atmosphérique et aux contraintes de cisaillement régnant sur la face avant du cylindre de coupe, dans la chambre de confinement et au niveau de la vis sans fin du tunnelier.

Ce fluide sous forme de mousse est également facilement pompable du fait de ces propriétés plastiques.

Il permet enfin d'améliorer la fluidité des déblais à base d'argile.

Les exemples suivants illustrent l'invention sans toutefois en limiter la portée.

## EXEMPLES

### Protocole général de préparation du gel

Le milieu apolaire (MApo) est placé dans un conteneur de 1 litre.

On y introduit l'agent "multicaténaire" (ATMC) à température ambiante, sous agitation à l'aide d'une pale déflocculeuse Rayneri® commercialisée par Rayneri, tournant à 400 tours/minute ; on agite encore pendant 5 minutes.

On introduit ensuite l'agent de neutralisation (AN) et on agite 5 minutes.

On ajoute enfin l'hydrocolloïde éventuel.

Après agitation pendant 5 minutes, on laisse reposer le milieu pendant 2 heures environ.

### Exemple 1 - Gel (G1-1) pour fluide de forage à base d'eau

On prépare selon le mode opératoire ci-dessus, le gel (G1-1) suivant :

Tableau 1

| Ingredients | Parties en poids | % en poids |
|---|---|---|
| huile de colza | 335 | 67 |
| LUBRHOPHOS LL550 ®* | 10 | 2 |
| aminométhyl propanol | 5 | 1 |
| RHODOPOL 23 ®** | 150 | 30 |

* tensio-actif "multicatenaire" à base de diester phosphate éthoxylé en $C_8$-$C_{14}$, présentant 5,5 motifs oxyéthylène, contenant de l'eau en quantité inférieure à 2% en poids, commercialisé par Rhône-Poulenc

** gomme xanthane commercialisée par Rhône-Poulenc

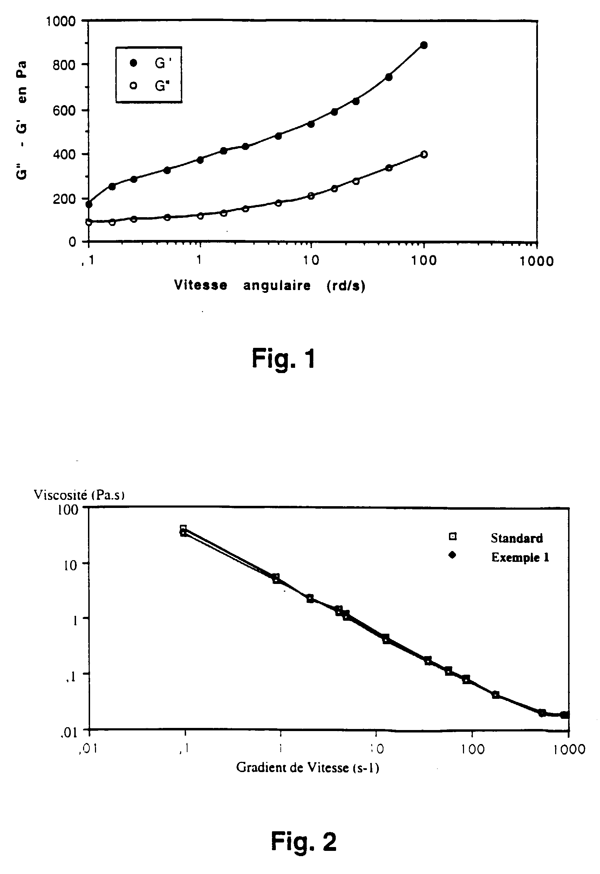

Le gel (G1-1) obtenu est un gel "souple". La courbe donnée à la figure 1 représente la variation du module de conservation G' et du module de déformation G" en fonction de la vitesse angulaire de déformation exprimée en radian/s.

Ce gel (G1-1) est ensuite dispersé dans l'eau, selon une quantité permettant d'obtenir un milieu dilué présentant une concentration en gomme xanthane de 0,5 % en poids.

La figure 2 montre que la variation de viscosité dynamique du milieu obtenu en fonction du gradient de vitesse, mesurée à l'aide du Rheomat 115® commercialisé par Contraves, est pratiquement semblable à celle d'une solution de gomme Xanthane à 0,5% dans l'eau.

La dispersion obtenue est donc pompable.

**Exemple 2 - Gel (G1-2) pour composition cosmétique**

On prépare selon le mode opératoire défini ci-dessus, le gel (G1-2) suivant :

Tableau 2

| Ingrédients | Parties en poids | % en poids |
|---|---|---|
| huile d'olive | 442,5 | 88,5 |
| RHODAFAC PC 100 ®* | 30 | 6 |
| aminométhyl propanol | 27,5 | 5,5 |

* tensio-actif "multicatenaire" à base d'un mélange de mono et dilaurylester phosphates éthoxylés contenant de l'eau en quantité inférieure à 2% en poids, commercialisé par Rhône-Poulenc

Le gel obtenu est "dur".

**Exemple 3 comparatif**

On prépare selon le mode opératoire défini ci-dessus, la composition suivante :

Tableau 3

| Ingrédients | % en poids |
|---|---|
| huile de colza | 87 |
| acide dodécylbenzènesulfonique | 5 |
| aminométhyl propanol | 8 |

L'utilisation dans la composition de l'acide dodécylbenzènesulfonique qui est un tensio-actif non multicaténaire ne permet pas d'obtenir un gel en milieu apolaire. La composition reste fluide.

**Exemple 4 - propriétés rhéologiques d'un fluide de forage préparé à l'aide d'un gel (G1-3) selon l'invention comprenant des hydrocolloïdes sous forme d'un mélange (M).**

Préparation d'un mélange (M)

On mélange selon un rapport pondéral de 80/20, un mélange d'hydroxypropylguar contenant 0,4 motif oxypropylène par molécule (JAGUAR HP8® de RHONE-POULENC) présentant un indice de pseudoplasticité en solution à 0,3% en poids dans de l'eau de dureté 22HT (324 mg/l de $CaCl_2$, 2 $H_2O$) de 0,94 et de RHEOZAN® (de RHONE-POULENC) présentant un indice de pseudoplasticité en solution à 0,1% en poids dans de l'eau de dureté 22HT (324 mg/l de $CaCl_2$, 2 $H_2O$) de 0,36.

Un gel (G1-3) est préparé comme dans l'exemple 1 si ce n'est que le RHODOPOL 23 ® est remplacé par le mélange (M).

Le gel (G1-3) est dispersé dans de l'eau, selon une quantité permettant d'obtenir un milieu dilué présentant une concentration en mélange (M) de 1 % en poids. On étudie les propriétés du fluide de forage obtenu à différents pH de 4,5, 9,6 et 13 et en présence ou non de ciment à des taux de concentration de 1 ou 5 % en poids.

Le gel (G1-3) et le ciment sont ajoutés dans l'eau 22HT sous agitation à l'aide d'un agitateur IKA à 1500 tr/min.

Les mesures à différents pH permettent de traduire le comportement du fluide de forage en contact avec des terrains plus ou moins acides. L'ajustage du pH se fait immédiatement après la dispersion du gel (G1-3) dans l'eau, puis on laisse la solution reposée 1 heure avant l'ajout éventuel de ciment (Portland CPA 55).

L'ajustage des pH se fait à l'aide d'acide acétique à 10 % pour le pH de 4,5 et à l'aide de soude à 10 % pour le pH de 13. Le pH de 9,6 correspond au pH naturel du gel (G1-3) comprenant le mélange (M).

Mesures réalisées

Pour des solutions de concentrations différentes, les valeurs de viscosité plastique ($V_p$), de yield value (YV) et de viscosité Marsh ($V_M$) sont mesurées à 0h, 2h ou 24h après préparation des différents fluides. les mesures sont réalisées selon les tests suivants.

a. Test pour la viscosité plastique ($V_p$) et la yield value ($Y_v$) :

- on prépare 400 g d'un fluide à base d'eau par dilution du gel (G1-3) dans l'eau : le gel est hydraté dans de l'eau 22HT à l'aide d'un agitateur IKA, la pâle défloculeuse tournant à une vitesse de 400 rpm lors de l'introduction du gel puis à 800 rpm pendant 15 min, puis
- on mesure la viscosité de la solution à l'aide d'un viscosimètre FANN à 300 et 600 rpm.
- on en déduit :

    . la viscosité plastique : $V_p = L_{600}-L_{300}$ en MPa.s, et
    . la yield value YV= $L_{600}/2 - V_p$ en Pa.

b. Test pour la viscosité Marsh ($V_M$) :

- on prépare 1000 g d'un fluide à base d'eau par dilution du gel (G1-3) dans l'eau : le mélange (M) est hydraté dans de l'eau 22HT à l'aide d'un agitateur IKA, la pâle défloculeuse tournant à une vitesse de 800 rpm lors de l'introduction du gel puis pendant 15 min,
- à l'aide d'un viscosimètre MARSH, on mesure le temps d'écoulement en secondes de 946 cc de solution.

Les résultats sont rassemblés dans le tableau 3.

Tableau 3

| Formulation | pH initial du gel (G1-3) | Age | $V_p$ | YV | $V_M$ |
|---|---|---|---|---|---|
| G1-3 | 9,6 | 2 h | 7 | 5 | 44 |
|  |  | 24 h | 7 | 5 | 46 |
| G1-3 | 4,5 | 2 h | 7 | 5 | 47 |
|  |  | 24 h | 7 | 5 | 47 |
| G1-3 | 13 | 2 h | 6 | 3 | 40 |
|  |  | 24 h | 6 | 3,5 | 40 |
| G1-3 ciment 1% | 9,6 | 0 h | 7 | 4 | 42 |
|  |  | 24 h | 7 | 4 | 42 |
| G1-3 ciment 1% | 4,5 | 0 h | 8 | 4 | 42 |
|  |  | 24 h | 7 | 3,5 | 43 |
| G1-3 ciment 1% | 13 | 0 h | 6 | 1,5 | 37 |
|  |  | 24 h | 5 | 2 | 42 |
| G1-3 ciment 5% | 9,6 | 0 h | 7 | 4 | 38 |
|  | 9,6 | 24 h | 6 | 3 | 30 |
| G1-3 ciment 5% | 4,5 | 0 h | 8 | 4 | 40 |
|  |  | 24 h | 6 | 4,5 | 32 |
| G1-3 ciment 5% | 13 | 0 h | 7 | 3,5 | 40 |
|  |  | 24 h | 6 | 4 | 38 |

On constate que les valeurs de la viscosité plastique ($V_p$), de la viscosité Marsh ($V_M$) et de la Yield Value (YV) sont peu affectées par la présence du ciment à des concentrations de 1 ou 5 % en poids.

On a également mesuré la valeur des filtrats afin d'évaluer la capacité du fluide à retenir l'eau. Pour cela, on filtre les fluides après contamination par ciment avec un filtre presse basse température (modèle 12 BL 2 Fann) et on mesure le volume de filtrat pour t = 1 min, 4 min, 7 min 30 s et 30 min, respectivement $V_1$, $V_4$, $V_{7,3}$ et $V_{30}$.

Les résultats sont rassemblés dans le tableau 4.

Tableau 4

| Formulation | pH initial du gel (G1-3) | Age | $V_1$ | $V_4$ | $V_{7,3}$ | $V_{30}$ |
|---|---|---|---|---|---|---|
| G1-3 | 4,5 | 2 h | 7,5 | 10 | 11,5 | 17 |
| G1-3 ciment 1% | 9,6 | 0 h | 4 | 7 | 8,5 | 15 |
| | | 24 h | 6 | 8,1 | 9,8 | 16,4 |
| G1-3 ciment 1% | 4,5 | 0 h | 4 | 7 | 8,2 | 14,8 |
| | | 24 h | 4,6 | 6,9 | 8,5 | 16 |
| G1-3 ciment 1% | 13 | 0 h | 2,4 | 4 | 5,6 | 12,4 |
| | | 24 h | 1 | 1,6 | 4,4 | 11,8 |
| G1-3 ciment 5% | 9,6 | 0 h | 6 | 12 | 15 | 24 |
| | | 24 h | 3 | 4,8 | 6,2 | 14 |
| G1-3 ciment 5% | 4,5 | 0 h | 5 | 11 | 14,6 | 24,8 |
| | | 24 h | 5 | 7 | 8,8 | 17 |
| G1-3 ciment 5% | 13 | 0 h | 5 | 8,5 | 11 | 19,8 |
| | | 24 h | 13 | 26 | 31 | 42 |

On observe que les valeurs de filtrat restent faibles même en présence de ciment. Le fluide de forage conserve sa propriété de rétention d'eau après utilisation

**Exemple 5 comparatif - bentonite**

On réalise des fluides de forage à base de bentonite et on les soumet aux mêmes tests que dans l'exemple 3. Les résultats sont rassemblés dans les tableaux 5 et 6.

Le pH naturel du fluide de forage à base de bentonite est de 10,7.

Tableau 5

| Formulation | pH initial du fluide de forage | Age | $V_p$ | YV | $V_M$ |
|---|---|---|---|---|---|
| bentonite | 10,7 | 2 h | 7 | 5 | 41 |
| bentonite | 4,5 | 2 h | 5 | 0 | 33 |
| bentonite | 13 | 2 h | 5 | 54 | **gel** |
| bentonite ciment 1% | 10,7 | 0 h | 7 | 24 | 58 |
| | | 24 h | **gel** | **gel** | **gel** |
| bentonite ciment 1% | 4,5 | 0 h | 7 | 7 | 36 |
| | | 24 h | 8 | 6,5 | **gel** |
| bentonite ciment 1% | 13 | 0 h | 11 | 6,5 | 38 |
| | | 24 h | 14 | 9 | **gel** |
| bentonite ciment 5% | 10,7 | 0 h | 17 | 15 | 45 |
| | | 24 h | **gel** | **gel** | **gel** |
| bentonite ciment 5% | 4,5 | 0 h | 7 | 5,5 | 36 |
| bentonite ciment 5% | 13 | 0 h | 25 | 15 | 240 |

On observe que la bentonite contaminée par le ciment gélifie au bout de 24 h. Ce gel n'est pas pompable et ne peut plus être utilisé comme fluide de forage pour la fabrication de parois moulées.

EP 0 714 978 B1

Tableau 6

| Formulation | pH | Age | $V_1$ | $V_4$ | $V_{7,3}$ | $V_{30}$ |
|---|---|---|---|---|---|---|
| bentonite | 4,5 | 2 h | 3 | 7 | 9 | 17 |
| bentonite ciment 1% | 10,7 | 0 h<br>24 h | 16<br>gel | 33,5<br>gel | 46,8<br>gel | 99,8<br>gel |
| bentonite ciment 1% | 4,5 | 0 h<br>24 h | 29,5<br>38 | 59,2<br>76 | 85,4<br>106 | gel<br>gel |
| bentonite ciment 1% | 13 | 0 h<br>24 h | 11<br>24 | 24<br>49 | 30,2<br>67 | 79,7<br>gel |
| bentonite ciment 5% | 10,7 | 0 h<br>24 h | 26,3<br>gel | 52,4<br>gel | 74<br>gel | gel<br>gel |
| bentonite ciment 5% | 4,5 | 0 h | 40 | 81 | 114 | gel |
| bentonite ciment 5% | 13 | 0 h | 17 | 34 | 47 | 102 |

On constate que dès que la bentonite est en présence d'ions $Ca^{2+}$, les valeurs des filtrats s'élèvent considérablement : la bentonite qui présentait de bonnes propriétés de rétention d'eau en l'absence de ciment ($V_{30}$ < 20 ml) n'assure plus cette fonction après une première utilisation en présence de ciment.

**Exemple 6 : système précurseur de fluide de forage sous forme de mousse, utilisation en milieu sablo-graveleux**

On introduit dans de l'eau :

.   le gel (G1-1) de l'exemple 1 à raison de 1 % en poids par rapport au poids d'eau,
.   et du Rhodapex AB-20 de Rhône-Poulenc en tant qu'agent tensio-actif (TA) à raison de 0,5 % en poids par rapport au poids d'eau. Le Rhodapex AB-20 est un alkyléthersulfate d'ammonium.

Puis on agite ce mélange à l'aide d'un agitateur à pâles et on laisse reposer quelques minutes la composition obtenue.
La composition est ensuite injectée dans un générateur de mousse et mélangée à un terrain sablo-graveleux saturé à 15 % en eau. La composition est introduite à raison de 2 % en poids par rapport au poids du terrain et de l'eau dudit terrain.
Afin de contrôler la fluidité du terrain mélangé à la mousse, on réalise une mesure au cône d'Abrams du terrain : celle-ci est de 15 cm. Cette mesure traduit le comportement plastique du terrain contenant l'agent moussant. On constate d'autre part que le milieu ne sédimente pas.
Ce terrain est donc facilement pompable et évacuable contrairement à un terrain sableux sans ajout de mousse qui présente une sédimentation rapide et aucun comportement plastique
On constate également que le terrain n'adhère pas sur les aciers : la mousse permet donc de diminuer les frottements avec la roue dentée.

**Exemple 7 : système précurseur de fluide de forage sous forme de mousse, utilsiation en milieu sableux**

On introduit dans de l'eau :

.   le gel (G1-1) de l'exemple 1 à raison de 1 % en poids par rapport au poids d'eau,
.   et du Rhodapex AB-20 en tant qu'agent tensio-actif (TA) à raison de 0,8 % en poids par rapport au poids d'eau, puis on agite ce mélange à l'aide d'un agitateur à pâles et on laisse reposer quelques minutes la composition obtenue.

La composition est ensuite injectée dans un générateur de mousse et mélangée à un terrain sableux, présentant une forte discontinuité granulaire comprise entre 0,1 et 0,3 mm et une teneur en eau de 16 %. La composition est introduite à raison de 3 % en poids par rapport au poids du terrain et de l'eau dudit terrain.

Afin de contrôler la fluidité du terrain mélangé à la mousse, on réalise une mesure au cône d'Abrams du terrain : celle-ci est de 15 cm. Cette mesure traduit le comportement plastique du terrain contenant l'agent moussant. On constate d'autre part que le milieu ne sédimente pas.

Ce terrain est donc facilement pompable et évacuable contrairement à un terrain sableux sans ajout de mousse qui présente une sédimentation rapide et aucun comportement plastique

On observe également que le terrain n'adhère pas sur les aciers : la mousse permet donc de diminuer les frottements avec la roue dentée.

**Exemple 8 : système précurseur de fluide de forage sous forme de mousse, utilisation en milieu argileux**

On introduit dans de l'eau :

. le gel (G1-1) à raison de 0,1 % en poids par rapport au poids d'eau,
. et du Rhodapex AB-20 à raison de 1,5 % en poids par rapport au poids d'eau, puis on agite ce mélange à l'aide d'un agitateur à pâles et on laisse reposer quelques minutes la composition obtenue.

La composition est ensuite injectée dans un générateur de mousse et mélangée à un terrain limoneux, présentant une teneur en argile comprise entre 15 et 18 % et une teneur en eau de 15 %. La composition est introduite à raison de 10 % en poids par rapport au poids du terrain.

Afin de contrôler la fluidité du terrain mélangé à la mousse, on réalise une mesure au cône d'Abrams du terrain : celle-ci est de 15 cm. Cette mesure traduit le comportement plastique du terrain contenant la mousse. On constate d'autre part que le milieu ne sédimente pas.

Ce terrain est donc facilement pompable et évacuable contrairement à un terrain sableux sans ajout de mousse qui présente une sédimentation rapide et aucun comportement plastique

On constate également que le terrain n'adhère pas sur les aciers : la mousse permet donc de diminuer les frottements avec la roue dentée.

**Revendications**

1. Gel d'un milieu apolaire caractérisé en ce qu'il contient :

   - au moins un milieu apolaire (MApo),
   - au moins un agent tensio-actif liquide "multicaténaire" sous forme acide (ATMC), soluble ou dispersable dans ledit milieu apolaire,
   - au moins un agent de neutralisation (AN) dudit agent tensio-actif "multicaténaire", présent en quantité correspondant à 2 à 30 fois, de préférence 4 à 20 fois, la quantité stoechiométriquement nécessaire à la neutralisation dudit agent tensio-actif "multicaténaire" sous forme acide (ATMC),
   - de 0,2 à 5, de préférence de 0,3 à 3 molécules d'eau par molécule d'agent tensio-actif liquide "multicaténaire" sous forme acide (ATMC),
     l'eau étant introduite au moins en partie par l'intermédiaire dudit agent tensio-actif "multicaténaire" sous forme acide (ATMC) et/ou par l'intermédiaire dudit agent de neutralisation (AN),
   - éventuellement au moins un agent émulsifiant susceptible d'émulsifier ledit gel dans l'eau ou dans un milieu aqueux,
   - et éventuellement au moins une charge solide soluble ou insoluble dans le milieu apolaire (MApo).

2. Gel selon la revendication 1, caractérisé en ce que le milieu apolaire (MApo) est choisi parmi les triglycérides d'acides gras saturés ou insaturés ayant au moins 12 atomes de carbone et de préférence de 14 à 20 atomes de carbone, les coupes pétrolières aromatiques, les composés terpéniques, les mélanges de diesters succinate/adipate/glutarate de diméthyle, dipropyle, diisobutyle, dibutyle, les hydrocarbures aliphatiques contenant au moins 6 atomes de carbone, les solvants aromatiques, les solvants chlorés et les huiles essentielles.

3. Gel selon la revendication 1 ou 2, caractérisé en ce que l'agent de neutralisation (AN) dudit tensio-actif "multicaténaire" (ATMC), est liquide et non soluble dans ledit milieu de suspension apolaire.

4. Gel selon la revendication 3, caractérisé en ce que l'agent tensio-actif "multicaténaire" (ATMC) est choisi parmi les tensio-actifs à base de :

- diesters phosphates d'alcools alkoxylés, présentant de 2 à 20, de préférence de 4 à 10 motifs oxyalkylène, de préférence oxyéthylène, et pour lesquels les alcools sont choisis parmi les alcools aliphatiques saturés ou insaturés, linéaires ou ramifiés en $C_6$-$C_{30}$, de préférence en $C_6$-$C_{20}$, ou
- diesters phosphates d'alkylphénols éthoxylés, présentant de 2 à 20, de préférence de 4 à 10 motifs oxyalkylène, de préférence oxyéthylène, et pour lesquels les alkylphénols sont choisis parmi ceux dont le radical alkyle est en $C_6$-$C_{30}$, de préférence en $C_6$-$C_{20}$.

5. Gel selon la revendication 4 caractérisé en ce que l'agent de neutralisation (AN) est choisi parmi les amines primaires, secondaires ou tertiaires et les alcanolamines.

6. Gel selon la revendication 5 caractérisé en ce que l'agent de neutralisation (AN) est choisi parmi la triéthanolamine, l'aminométhylpropanol, la cocoamine, la butylamine.

7. Gel selon l'une quelconque des revendications 1 à 6, caractérisé en ce que l'agent susceptible d'émulsifier ledit gel dans l'eau ou dans un milieu aqueux, est choisi parmi les tristyrylphénols éthoxylés, les sulfates ou phosphates de tristyrylphénols éthoxylés et/ou propoxylés, les acides ou les alcools gras éthoxylés et/ou propoxylés, les copolymères blocs éthoxylés/propoxylés, le dodécylbenzène sulfonate de sodium.

8. Gel selon l'une quelconque des revendications 1 à 7, caractérisé en ce que les charges solides sont choisies parmi la silice, le carbonate de calcium, les pigments, les hydrocolloïdes susceptibles d'épaissir les milieux aqueux, les agents anti-mousse, les composés phytosanitaires, les métasilicates de sodium, les argiles, l'oxyde de titane, l'oxyde de zinc.

9. Gel selon la revendications 8, caractérisé en ce que les hydrocolloïdes susceptibles d'épaissir les milieux aqueux sont choisis parmi :

   . les polysaccharides obtenus par fermentation bactérienne,
   . les galactomannanes,
   . les carraghénanes,
   . les alginates,
   . les dérivés hémisynthétiques de la cellulose,
   . les polyacrylates de métaux alcalins,
   . les alcools polyvinyliques,
   . les polyéthylène glycols,
   . les polyvinylpyrrolidones,

   seuls ou en association entre eux.

10. Gel selon l'une quelconque des revendications précédentes, caractérisé en ce que la quantité d'agent tensio-actif liquide "multicaténaire" (ATMC) est de 0,5 à 10%, de préférence de 1 à 6%, en poids par rapport audit gel.

11. Gel selon l'une quelconque des revendications précédentes, caractérisé en ce qu'il est "souple" (ou "faible"), en ce que la quantité de tensio-actif "multicaténaire" (ATMC) est de 1 à 3% en poids par rapport au poids dudit gel, avec un rapport molaire agent de neutralisation (AN) / tensio-actif "multicaténaire" (ATMC) de 5 à 10 fois le rapport stoechiométrique de neutralisation, et en ce que la quantité d'eau est de 0,01 à 0,1% en poids par rapport au poids dudit gel.

12. Gel selon l'une quelconque des revendications 1 à 10, caractérisé en ce qu'il est "dur" (ou "fort"), en ce que la quantité de tensio-actif "multicaténaire" (ATMC) est de 5 à 10% en poids par rapport au poids dudit gel, avec un rapport molaire agent de neutralisation (AN) / tensio-actif "multicaténaire" (ATMC) de 5 à 10 fois le rapport stoechiométrique de neutralisation, et en ce que la quantité d'eau est de 0,05 à 0,5% en poids par rapport au poids dudit gel.

13. Gel (gel G1) selon l'une quelconque des revendications 1 à 9, caractérisé en qu'il comprend :

- 100 - (a+b+c+d) parties en poids d'au moins un milieu apolaire (MApo),

- une quantité (a) de 0,5 à 6, de préférence de 1 à 4 parties, en poids d'au moins un agent tensio-actif liquide "multicaténaire" sous forme acide (ATMC), soluble ou dispersable dans ledit milieu apolaire,
- une quantité (b) d'au moins un agent de neutralisation (AN) dudit agent tensio-actif "multicaténaire", correspondant à 2 à 30 fois, de préférence 4 à 20 fois, la quantité stoechiométriquement nécessaire à la neutralisation dudit agent tensio-actif "multicaténaire" sous forme acide (ATMC),
- une quantité (c) d'eau correspondant à 0,2 à 5, de préférence 0,3 à 3, molécules d'eau par molécule d'agent tensio-actif liquide "multicaténaire" sous forme acide (ATMC),
  l'eau étant introduite au moins en partie par l'intermédiaire dudit agent tensio-actif "multicaténaire" sous forme acide (ATMC) et/ou par l'intermédiaire dudit agent de neutralisation (AN),
- et une quantité (d) de 10 à 50, de préférence de 20 à 40 parties, en poids d'une charge constituée d'au moins un hydrocolloïde susceptible d'épaissir les milieux aqueux.

**14.** Gel (gel G1) selon la revendication 13, caractérisé en ce que l'hydrocolloïde susceptible d'épaissir les milieux aqueux est choisi parmi la gomme xanthane, les polysuccinoglycanes, le rhamsan, le wellan, le gellan.

**15.** Gel (gel G1) selon la revendication 13, caractérisé en ce que l'hydrocolloïde susceptible d'épaissir les milieux aqueux est un mélange (M) contenant :

- au moins un polysaccharide hydrocolloïde obtenu par fermentation bactérienne (PSB), stable en présence d'ions $Ca^{2+}$ à un pH supérieur à 10, et présentant un indice de pseudoplasticité inférieur ou égal à 0,5 à une concentration de 0,1% en poids dans l'eau distillée,
- et au moins un polymère hydrocolloïde d'origine naturelle (PN), stable en présence de ciment et d'ions $Ca^{2+}$ à un pH supérieur à 10, et présentant un indice de pseudoplasticité supérieur ou égal à 0,6 à une concentration de 0,3% en poids dans l'eau distillée,

selon un rapport pondéral PN / PSB de 20/80 à 95/5, de préférence de 50/50 à 90/10.

**16.** Gel (gel G1) selon la revendication 15, caractérisé en ce que le polysaccharide hydrocolloïde obtenu par fermentation bactérienne (PSB) est un polysuccinoglycane, une gomme rhamsan, une gomme welan.

**17.** Gel (gel G1) selon la revendication 15 ou 16, caractérisé en ce que le polymère hydrocolloïde d'origine naturelle (PN) est un dérivé hémisynthétique de la cellulose, un dérivé alcoxylé de la gomme guar contenant par molécule de 0,01 à 5, de préférence de 0,05 à 0,5, motif(s) oxyalkylène.

**18.** Gel (gel G1) selon la revendication 17, caractérisé en ce que le polymère hydrocolloïde d'origine naturelle (PN) est une hydroxyméthylcellulose, une hydroxyéthylcellulose, une hydroxyméthylpropylcellulose, une hydroxypropylcellulose, une carboxyméthylcellulose, une hydroxyalkylguar contenant par molécule de 0,01 à 5, de préférence de 0,05 à 0,5, motif(s) oxyéthylène et/ou oxypropylène.

**19.** Gel selon l'une des revendications 15 à 18 caractérisé en ce que le mélange (M) contient :

- un polysaccharide succinoglycane
- et un hydroxyalkylguar contenant de 0,01 à 5, de préférence de 0,05 à 0,5, motif(s) oxyéthylène et / ou oxypropylène

selon un rapport pondéral hydroxyalkylguar / succinoglycane de 20/80 à 95/5, de préférence de 50/50 à 90/10.

**20.** Procédé pour gélifier un milieu apolaire (MApo) caractérisé en ce qu'on introduit dans ledit milieu apolaire :

- au moins un agent tensio-actif liquide "multicaténaire" sous forme acide (ATMC), soluble ou dispersable dans ledit milieu apolaire,
- au moins un agent de neutralisation (AN) dudit agent tensio-actif "multicaténaire", présent en quantité correspondant à 2 à 30 fois, de préférence 4 à 20 fois, la quantité stoechiométriquement nécessaire à la neutralisation dudit agent tensio-actif "multicaténaire" sous forme acide (ATMC),
- de 0,2 à 5, de préférence de 0,3 à 3, molécules d'eau par molécule d'agent tensio-actif liquide "multicaténaire" sous forme acide (ATMC),
  l'eau étant introduite au moins en partie par l'intermédiaire dudit agent tensio-actif "multicaténaire" sous forme acide (ATMC) et/ou par l'intermédiaire dudit agent de neutralisation (AN),

- éventuellement au moins un agent émulsifiant susceptible d'émulsifier le gel obtenu dans l'eau ou dans un milieu aqueux,
- et éventuellement au moins une charge solide soluble ou insoluble dans le milieu apolaire (MApo).

21. Procédé selon la revendication 20, caractérisé en ce qu'il consiste à introduire sous agitation mécanique le tensio-actif "multicaténaire" (ATMC) dans le milieu apolaire (MApo), puis l'agent émulsifiant éventuel, l'agent de neutralisation (AN) et les charges éventuelles.

22. Procédé selon la revendication 20 ou 21, caractérisé en ce que les différents composés mis en oeuvre sont choisis parmi ceux entrant dans la composition du gel faisant l'objet de l'une quelconque des revendications 2 à 9 et 14 à 19.

23. Procédé selon l'une quelconque des revendications 20 à 22, caractérisé en ce que les différents composés sont mis en oeuvre selon des quantités correspondant aux quantités respectives des constituants du gel faisant l'objet de l'une quelconque des revendications 13 à 16.

24. Utilisation du gel défini selon l'une quelconque des revendications 13 à 19 pour la préparation d'un fluide de forage à base d'eau.

25. Utilisation selon la revendication précédente pour la préparation d'un fluide de forage à base d'eau pour la fabrication de parois moulées.

26. Procédé de préparation d'un fluide de forage à base d'eau, caractérisé en ce qu'on dilue dans l'eau le gel (gel G1) défini selon l'une des revendications 13 à 19 de telle manière que le fluide comprenne 0,01 à 3 partie(s) en poids d'hydrocolloïde susceptible d'épaissir les milieux aqueux, introduit par l'intermédiaire du gel (gel G1), de préférence de 0,05 à 1,5 partie en poids, pour 100 parties d'eau.

27. Utilisation du gel (gel G1) défini selon l'une des revendications 13 à 19 en association avec un agent tensio-actif (TA) comme système précurseur d'un fluide de forage sous forme de mousse.

28. Système précurseur d'un fluide de forage sous forme de mousse caractérisé en ce qu'il comprend le gel (gel G1) défini selon l'une des revendications 13 à 19 et un agent tensio-actif (TA).

29. Système précurseur d'un fluide de forage sous forme de mousse selon la revendication 28 caractérisé en ce que l'agent tensio-actif (TA) est choisi parmi :

.  les alkylsulfates de formule $ROSO_3M$, où R représente un radical alkyle ou hydroxyalkyle en $C_5$-$C_{24}$, de préférence en $C_{10}$-$C_{18}$, M représentant un atome d'hydrogène ou un cation alcalin (sodium, potassium, lithium), ammonium substitué ou non substitué (méthyl-, diméthyl-, triméthyl-, tetraméthylammonium, diméthylpiperidinium) ou dérivé d'une alcanolamine (monoéthanolamine, diéthanolamine, triéthanolamine, monoisopropanolamine) ;
.  les bétaïnes, les alkylbétaïnes, les alkyldiméthylbétaïnes, les alkylamidobétaïnes, les alkyltriméthylsulfobétaïnes ;
.  les mélanges d'alkylamidobétaïnes et d'alkylsulfosuccinates de sodium où le radical alkyle est en $C_4$- $C_{18}$ ;
.  les alkyléthersulfates $RO(CH_2CHR_1O)_nSO_3M$ où n est compris entre 0,5 et 30 , de préférence entre 0,5 et 10, et où $R_1$ est un groupement alkyl en $C_4$-$C_{18}$, et présentant en moyenne de 0,5 à 30 motifs, de préférence de 0,5 à 10 motifs oxyéthylène et/ou oxypropylène, M représentant un atome d'hydrogène ou un cation alcalin (sodium, potassium, lithium), ammonium substitué ou non substitué (méthyl-, diméthyl-, triméthyl-, tetraméthylammonium, diméthylpiperidinium,) ou dérivé d'une alcanolamine (monoéthanolamine, diéthanolamine, triéthanolamine, monoisopropanolamine) ;
.  les alpha oléfines sulfonates ;
.  les alkylpolyglycosides de formule $R(OCH_2CH_2)_n(G)_m$, où R est un radical hydrocarboné, linéaire ou ramifié, saturé ou insaturé en $C_8$-$C_{24}$, G est un sucre réducteur en $C_5$-$C_6$ tels le galactose, le glucose ou le fructose, n étant compris entre 0 et 20 et m entre 1 et 10,
.  les alkylamphomonoacétates, les alkylamphodiacétates tels que le cocoamphoacétate de sodium.

30. Système précurseur d'un fluide de forage sous forme de mousse selon l'une des revendications 28 ou 29, caractérisé en ce que la teneur en tensio-actif (TA) est comprise entre 1 et 2000 % en poids par rapport au poids du gel (gel G1).

**31.** Système précurseur d'un fluide de forage sous forme de mousse selon l'une quelconque des revendications 28 à 30, caractérisé en ce qu'il comprend en outre au moins une charge solide choisie parmi :

- . la silice naturelle ou synthétique,
- . le carbonate de calcium, le carbonate de magnésium,
- . les oxydes métalliques tels que l'oxyde de fer,
- . les pigment naturels ou synthétiques,
- . des agents régulateurs de mousse tels que le stéarate d'aluminium, la silice hydrophobe, l'éthylène bis stéaramide,
- . la chaux naturelle, éteinte, hydroïque,
- . le gypse,
- . des matières actives comme des composés phytosanitaires,
- . des composés basiques solubles dans l'eau tels que métasilicates de sodium, polysilicates, argiles, talcs, mica.

**32.** Système précurseur d'un fluide de forage sous forme de mousse selon la revendication 31, caractérisé en ce que la quantité de charge(s) solide représente au plus 50 % en poids dudit système précurseur, de préférence au plus 20 %, encore plus préférentiellement au plus 15 %.

**33.** Procédé de préparation d'un fluide de forage sous forme de mousse par dilution dans l'eau du système précurseur défini selon l'une des revendications 28 à 32 et agitation de telle manière que la quantité finale dans l'eau de l'agent tensio-actif (TA) du système précurseur est comprise entre 0,05 et 25 % en poids par rapport à l'eau, de préférence entre 0,05 et 15 %, encore plus préférentiellement entre 0,2 et 1,5 %.

**34.** Utilisation du fluide de forage sous forme de mousse obtenu à l'aide du système précurseur défini selon l'une quelconque des revendications 28 à 32 ou par le procédé défini selon la revendication 33 pour l'excavation de tunnels.

**Patentansprüche**

**1.** Gel eines nichtpolaren Mediums, dadurch gekennzeichnet, daß es enthält:

- - mindestens ein nichtpolares Medium (MApo),
- - mindestens ein "multikettenförmiges" flüssiges oberflächenaktives Mittel in der sauren Form (ATMC), das in dem genannten nichtpolaren Medium löslich oder dispergierbar ist,
- - mindestens ein Neutralisierungsmittel (AN) des "multikettenförmigen" oberflächenaktiven Mittels, anwesend in einer Menge, die dem Zwei- bis Dreißigfachen, vorzugsweise dem Vier- bis Zwanzigfachen der für die Neutralisation des "multikettenförmigen" oberflächenaktiven Mittels in der sauren Form (ATMC) stöchiometrisch notwendigen Menge entspricht,
- - 0,2 bis 5, vorzugsweise 0,3 bis 3 Moleküle Wasser pro Molekül des "multikettenförmigen" flüssigen oberflächenaktiven Mittels in der sauren Form (ATMC),
  wobei das Wasser mindestens teilweise durch Vermittlung des genannten "multikettenförmigen" oberflächenaktiven Mittels in der sauren Form (ATMC) und/oder durch Vermittlung des genannten Neutralisierungsmittels (AN) eingebracht wird,
- - gegebenenfalls mindestens ein Emulgierungsmittel, das geeignet ist, das genannte Gel in Wasser oder in einem wäßrigen Medium zu emulgieren, und
- - gegebenenfalls mindestens einen festen Füllstoff, der in dem nichtpolaren Medium (MApo) löslich oder unlöslich ist.

**2.** Gel nach Anspruch 1, dadurch gekennzeichnet, daß das nichtpolare Medium (MApo) unter den Triglyceriden von gesättigten oder ungesättigten Fettsäuren mit mindestens 12 Kohlenstoffatomen und vorzugsweise 14 bis 20 Kohlenstoffatomen, den aromatischen Schnitten von Erdöl, den Terpen-Verbindungen, den Mischungen von Diestern Succinat/Adipat/Glutarat von Dimethyl, Dipropyl, Diisobutyl, Dibutyl, den aliphatischen Kohlenwasserstoffen mit mindestens 6 Kohlenstoffatomen, den aromatischen Lösungsmitteln, den chlorierten Lösungsmitteln und den essentiellen Ölen ausgewählt wird.

**3.** Gel nach Anspruch 1 oder 2, dadurch gekennzeichnet, daß das Neutralisierungsmittel (AN) des "multikettenförmigen" oberflächenaktiven Mittels (ATMC) flüssig und in dem genannten Medium der nichtpolaren Suspension unlöslich ist.

4. Gel nach Anspruch 3, dadurch gekennzeichnet, daß das "multikettenförmige" oberflächenaktive Mittel (ATMC) ausgewählt wird unter den oberflächenaktiven Mitteln auf der Basis von:

- Diester-phosphaten von alkoxylierten Alkoholen mit 2 bis 20, vorzugsweise 4 bis 10 Struktureinheiten Oxyalkylen, vorzugsweise Oxyethylen, wobei die Alkohole unter den gesättigten oder ungesättigten, geraden oder verzweigten aliphatischen Alkoholen mit 6 bis 30, vorzugsweise 6 bis 20 Kohlenstoffatomen ausgewählt werden, oder
- Diester-phosphaten von ethoxylierten Alkylphenolen mit 2 bis 20, vorzugsweise 4 bis 10 Struktureinheiten Oxyalkylen, vorzugsweise Oxyethylen, wobei die Alkylphenole unter denjenigen ausgewählt werden, deren Rest Alkyl 6 bis 30, vorzugsweise 6 bis 20 Kohlenstoffatome umfaßt.

5. Gel nach Anspruch 4, dadurch gekennzeichnet, daß das Neutralisierungsmittel (AN) unter den primären, sekundären oder tertiären Aminen und den Alkanolaminen ausgewählt wird.

6. Gel nach Anspruch 5, dadurch gekennzeichnet, daß das Neutralisierungsmittel (AN) unter Triethanolamin, Aminomethylpropanol, Cocoamin und Butylamin ausgewählt wird.

7. Gel nach irgendeinem der Ansprüche 1 bis 6, dadurch gekennzeichnet, daß das für das Emulgieren des genannten Gels in Wasser oder in einem wäßrigen Medium geeignete Mittel unter den ethoxylierten Tristyrylphenolen, den Sulfaten oder Phosphaten von ethoxylierten und/oder propoxylierten Tristyrylphenolen, den ethoxylierten und/oder propoxylierten Fettsäuren oder Fettalkoholen, den ethoxylierten und/oder propoxylierten Block-Copolymeren und dem Natrium-Dodecylbenzolsulfonat ausgewählt wird.

8. Gel nach irgendeinem der Ansprüche 1 bis 7, dadurch gekennzeichnet, daß die festen Füllstoffe unter Siliciumdioxid, Calciumcarbonat, den Pigmenten, den für das Eindicken der wäßrigen Medien geeigneten Hydrokolloiden, den Antischaummitteln, den Pflanzenschutzmitteln, den Natriummetasilicaten, den Tonen, Titanoxid und Zinkoxid ausgewählt werden.

9. Gel nach Anspruch 8, dadurch gekennzeichnet, daß die für das Eindicken der wäßrigen Medien geeigneten Hydrokolloide ausgewählt werden unter:

- den Polysacchariden, erhalten durch bakterielle Fermentation,
- den Galactomannanen,
- den Carraghenanen,
- den Alginaten,
- den halbsynthetischen Derivaten von Cellulose,
- den Polyacrylaten von Alkalimetallen,
- den Polyvinylalkoholen,
- den Polyethylenglycolen,
- den Polyvinylpyrrolidonen,

allein oder in Assoziation untereinander.

10. Gel nach irgendeinem der vorstehenden Ansprüche, dadurch gekennzeichnet, daß die Menge des "multikettenförmigen" flüssigen oberflächenaktiven Mittels (ATMC) 0,5 Gew.-% bis 10 Gew.-%, vorzugsweise 1 Gew.-% bis 6 Gew.-% beträgt, bezogen auf das genannte Gel.

11. Gel nach irgendeinem der vorstehenden Ansprüche, dadurch gekennzeichnet, daß es "weich" (oder "schwach") ist und daß die Menge des "multikettenförmigen" oberflächenaktiven Mittels (ATMC) 1 Gew.-% bis 3 Gew.-% beträgt, bezogen auf das Gewicht des genannten Gels, mit einem molaren Verhältnis Neutralisierungsmittel (AN)/ "multikettenförmiges" oberflächenaktives Mittel (ATMC) von fünf- bis zehnmal dem stöchiometrischen Verhältnis der Neutralisation, und daß die Menge an Wasser 0,01 Gew.-% bis 0,1 Gew.-% beträgt, bezogen auf das Gewicht des genannten Gels.

12. Gel nach irgendeinem der Ansprüche 1 bis 10, dadurch gekennzeichnet, daß es "hart" (oder "stark") ist und daß die Menge des "multikettenförmigen" oberflächenaktiven Mittels (ATMC) 5 Gew.-% bis 10 Gew.-% beträgt, bezogen auf das Gewicht des genannten Gels, mit einem molaren Verhältnis Neutralisierungsmittel (AN)/"multikettenförmiges" oberflächenaktives Mittel (ATMC) von fünf- bis zehnmal dem stöchiometrischen Verhältnis der Neutralisation, und daß die Menge an Wasser 0,05 Gew.-% bis 0,5 Gew.-% beträgt, bezogen auf das Gewicht des genannten Gels.

**13.** Gel (Gel G1) nach irgendeinem der Ansprüche 1 bis 9, dadurch gekennzeichnet, daß es umfaßt:

- 100-(a+b+c+d) Gewichtsteile von mindestens einem nichtpolaren Medium (MApo),
- eine Menge (a) von 0,5 bis 6, vorzugsweise 1 bis 4 Gewichtsteilen von mindestens einem "multikettenförmigen" oberflächenaktiven Mittel in der sauren Form (ATMC), das in dem genannten nichtpolaren Medium löslich oder dispergierbar ist,
- eine Menge (b) von mindestens einem Neutralisierungsmittel (AN) des genannten "multikettenförmigen" oberflächenaktiven Mittels, die dem Zwei- bis Dreißigfachen, vorzugsweise dem Vier- bis Zwanzigfachen der für die Neutralisation des "multikettenförmigen" oberflächenaktiven Mittels in der sauren Form (ATMC) stöchiometrisch notwendigen Menge entspricht,
- eine Menge (c) von Wasser, die 0,2 bis 5, vorzugsweise 0,3 bis 3 Molekülen Wasser pro Molekül des "multikettenförmigen" flüssigen oberflächenaktiven Mittels in der sauren Form (ATMC) entspricht, wobei das Wasser mindestens teilweise durch Vermittlung des genannten "multikettenförmigen" oberflächenaktiven Mittels in der sauren Form (ATMC) und/oder durch Vermittlung des genannten Neutralisierungsmittels (AN) eingebracht wird, und
- eine Menge (d) von 10 bis 50, vorzugsweise 20 bis 40 Gewichtsteilen eines Füllstoffes, bestehend aus mindestens einem Hydrokolloid, das geeignet ist, die wäßrigen Medien einzudicken.

**14.** Gel (Gel G1) nach Anspruch 13, dadurch gekennzeichnet, daß das für das Eindicken der wäßrigen Medien geeignete Hydrokolloid unter Xanthangummi, den Polysuccinoglycanen, Rhamsan, Wellan und Gellan ausgewählt wird.

**15.** Gel (Gel G1) nach Anspruch 13, dadurch gekennzeichnet, daß das für das Eindicken der wäßrigen Medien geeignete Hydrokolloid eine Mischung (M) ist, die enthält:

- mindestens ein Polysaccharid-Hydrokolloid (PSB), erhalten durch bakterielle Fermentation, das in Anwesenheit von Ionen $Ca^{2+}$ bei einem pH-Wert von über 10 stabil ist, und das einen pseudoplastischen Index von unterhalb oder gleich 0,5 bei einer Konzentration von 0,1 Gew.-% in destilliertem Wasser aufweist, und
- mindestens ein Polymer-Hydrokolloid (PN) natürlichen Ursprungs, das in Anwesenheit von Zement und Ionen $Ca^{2+}$ bei einem pH-Wert von über 10 stabil ist, und das einen pseudoplastischen Index von oberhalb oder gleich 0,6 bei einer Konzentration von 0,3 Gew.-% in destilliertem Wasser aufweist,

gemäß einem Gewichtsverhältnis PN/PSB von 20/80 bis 95/5, vorzugsweise von 50/50 bis 90/10.

**16.** Gel (Gel G1) nach Anspruch 15, dadurch gekennzeichnet, daß das durch bakterielle Fermentation erhaltene Polysaccharid-Hydrokolloid (PSB) ein Polysuccinoglycan, ein Rhamsan-Gummi oder ein Welan-Gummi ist.

**17.** Gel (Gel G1) nach Anspruch 15 oder 16, dadurch gekennzeichnet, daß das Polymer-Hydrokolloid (PN) natürlichen Ursprungs ein halbsynthetisches Derivat von Cellulose oder ein alkoxyliertes Derivat von Guar-Gummi ist, das pro Molekül 0,01 bis 5, vorzugsweise 0,05 bis 0,5 Struktureinheit(en) Oxyalkylen enthält.

**18.** Gel (Gel G1) nach Anspruch 17, dadurch gekennzeichnet, daß das Polymer-Hydrokolloid (PN) natürlichen Ursprungs eine Hydroxymethylcellulose, eine Hydroxyethylcellulose, eine Hydroxymethylpropylcellulose, eine Hydroxypropylcellulose, eine Carboxymethylcellulose oder ein Hydroxyalkyl-Guar ist, das pro Molekül 0,01 bis 5, vorzugsweise 0,05 bis 0,5 Struktureinheit(en) Oxyethylen und/oder Oxypropylen enthält.

**19.** Gel nach irgendeinem der Ansprüche 15 bis 18, dadurch gekennzeichnet, daß die Mischung (M) enthält:

- ein Succinoglycan-Polysaccharid und
- ein Hydroxyalkyl-Guar, das 0,01 bis 5, vorzugsweise 0,05 bis 0,5 Struktureinheit(en) Oxyethylen und/oder Oxypropylen enthält, gemäß einem Gewichtsverhältnis Hydroxyalkyl-Guar/Succinoglycan von 20/80 bis 95/5, vorzugsweise von 50/50 bis 90/10.

**20.** Verfahren zum Gelieren eines nichtpolaren Mediums (MApo), dadurch gekennzeichnet, daß man in das genannte nichtpolare Medium einträgt:

- mindestens ein "multikettenförmiges" flüssiges oberflächenaktives Mittel in der sauren Form (ATMC), das in dem genannten nichtpolaren Medium löslich oder dispergierbar ist,
- mindestens ein Neutralisierungsmittel (AN) des "multikettenförmigen" oberflächenaktiven Mittels, anwesend in einer Menge, die dem Zwei- bis Dreißigfachen, vorzugsweise dem Vier- bis Zwanzigfachen der für die Neutralisation des genannten "multikettenförmigen" oberflächenaktiven Mittels in der sauren Form (ATMC) stöchiometrisch notwendigen Menge entspricht,
- 0,2 bis 5, vorzugsweise 0,3 bis 3 Moleküle Wasser pro Molekül des "multikettenförmigen" flüssigen oberflächenaktiven Mittels in der sauren Form (ATMC), wobei das Wasser mindestens teilweise durch Vermittlung des genannten "multikettenförmigen" oberflächenaktiven Mittels in der sauren Form (ATMC) und/oder durch Vermittlung des genannten Neutralisierungsmittels (AN) eingebracht wird,
- gegebenenfalls mindestens ein Emulgierungsmittel, das geeignet ist, das erhaltene Gel in Wasser oder in einem wäßrigen Medium zu emulgieren, und
- gegebenenfalls mindestens einen festen Füllstoff, der in dem nichtpolaren Medium (MApo) löslich oder unlöslich ist.

**21.** Verfahren nach Anspruch 20, dadurch gekennzeichnet, daß es darin besteht, das "multikettenförmige" oberflächenaktive Mittel (ATMC) unter mechanischem Rühren in das nichtpolare Medium (MApo) einzutragen sowie anschließend gegebenenfalls das Emulgierungsmittel, das Neutralisationsmittel (AN) und gegebenenfalls die Füllstoffe.

**22.** Verfahren nach Anspruch 20 oder 21, dadurch gekennzeichnet, daß die verschiedenen eingesetzten Verbindungen unter denen ausgewählt werden, die in die Zusammensetzung des Gels, das den Gegenstand von irgendeinem der Ansprüche 2 bis 9 und 14 bis 19 bildet, Eingang finden.

**23.** Verfahren nach irgendeinem der Ansprüche 20 bis 22, dadurch gekennzeichnet, daß die verschiedenen Verbindungen gemäß den Mengen eingesetzt werden, die den jeweiligen Mengen der Bestandteile des Gels entsprechen, das den Gegenstand von irgendeinem der Ansprüche 13 bis 16 bildet.

**24.** Verwendung des Gels wie nach irgendeinem der Ansprüche 13 bis 19 definiert zur Herstellung einer Bohrflüssigkeit auf Wasserbasis.

**25.** Verwendung nach dem vorstehenden Anspruch zur Herstellung einer Bohrflüssigkeit auf Wasserbasis für die Fertigung von geformten Wandungen.

**26.** Verfahren zur Herstellung einer Bohrflüssigkeit auf Wasserbasis, dadurch gekennzeichnet, daß man das Gel (Gel G1), wie in einem der Ansprüche 13 bis 19 definiert, in der Weise in Wasser verdünnt, daß die Flüssigkeit 0,01 bis 3 Gewichtsteil(e) Hydrokolloid, das zum Eindicken der wäßrigen Medien geeignet ist, eingebracht durch Vermittlung des Gels (Gel G1), vorzugsweise 0,05 bis 1,5 Gewichtsteile, pro 100 Teile Wasser umfaßt.

**27.** Verwendung des Gels (Gel G1) wie nach irgendeinem der Ansprüche 13 bis 19 definiert in Assoziation mit einem oberflächenaktiven Mittel (TA) als Vorläufersystem einer Bohrflüssigkeit in Form von Schaum.

**28.** Vorläufersystem einer Bohrflüssigkeit in Form von Schaum, dadurch gekennzeichnet, daß es das Gel (Gel G1), wie nach einem der Ansprüche 13 bis 19 definiert, und ein oberflächenaktives Mittel (TA) umfaßt.

**29.** Vorläufersystem einer Bohrflüssigkeit in Form von Schaum nach Anspruch 28, dadurch gekennzeichnet, daß das oberflächenaktive Mittel (TA) ausgewählt wird unter:

. den Alkylsulfaten der Formel $ROSO_3M$, worin R einen Rest Alkyl oder Hydroxyalkyl mit 5 bis 24 Kohlenstoffatomen, vorzugsweise 10 bis 18 Kohlenstoffatomen darstellt, M ein Wasserstoffatom oder ein Alkali-Kation (Natrium, Kalium, Lithium), Ammonium-Kation, substituiert oder nicht substituiert (Methyl-, Dimethyl-, Trimethyl-, Tetramethylammonium, Dimethylpiperidinium) oder ein Derivat eines Alkanolamins (Monoethanolamin, Diethanolamin, Triethanolamin, Monoisopropanolamin) bedeutet;
. den Betainen, den Alkylbetainen, den Alkyldimethylbetainen, den Alkylamidobetainen, den Alkyltrimethylsulfobetainen,

. den Mischungen von Alkylamidobetainen und Natrium-Alkylsulfosuccinaten, worin der Rest Alkyl 4 bis 18 Kohlenstoffatome aufweist,

. den Alkylethersulfaten $RO(CH_2CHR_1O)_nSO_3M$, worin n zwischen 0,5 und 30, vorzugsweise zwischen 0,5 und 10 beträgt, und worin $R_1$ eine Gruppe Alkyl mit 4 bis 18 Kohlenstoffatomen darstellt, wobei diese Verbindungen im Mittel 0,5 bis 30, vorzugsweise 0,5 bis 10 Struktureinheiten Oxyethylen und/oder Oxypropylen aufweisen, und M ein Wasserstoffatom oder ein Alkali-Kation (Natrium, Kalium, Lithium), Ammonium-Kation, substituiert oder nicht substituiert (Methyl-, Dimethyl-, Trimethyl-, Tetramethylammonium, Dimethylpiperidinium) oder ein Derivat eines Alkanolamins (Monoethanolamin, Diethanolamin, Triethanolamin, Monoisopropanolamin) bedeutet;

. den alpha-Olefinsulfonaten;

. den Alkylpolyglycosiden der Formel $R(OCH_2CH_2)_n(G)_m$, worin R ein gerader oder verzweigter, gesättigter oder ungesättigter Kohlenwasserstoffrest mit 8 bis 24 Kohlenstoffatomen ist, G einen reduzierenden Zucker mit 5 bis 6 Kohlenstoffatomen wie Galactose, Glucose oder Fructose bedeutet, n zwischen 0 und 20 und m zwischen 1 und 10 betragen;

. den Alkylamphomonoacetaten, den Alkylamphodiacetaten wie Natrium-Cocoamphoacetat.

30. Vorläufersystem einer Bohrflüssigkeit in Form von Schaum nach einem der Ansprüche 28 oder 29, dadurch gekennzeichnet, daß der Gehalt des oberflächenaktiven Mittels (TA) zwischen 1 Gew.-% und 2000 Gew.-% liegt, bezogen auf das Gewicht des Gels (Gel G1).

31. Vorläufersystem einer Bohrflüssigkeit in Form von Schaum nach einem der Ansprüche 28 bis 30, dadurch gekennzeichnet, daß es außerdem mindestens einen festen Füllstoff umfaßt, ausgewählt unter:

. natürlichem oder synthetischem Siliciumdioxid,

. Calciumcarbonat, Magnesiumcarbonat,

. Metalloxiden wie Eisenoxid,

. natürlichen oder synthetischen Pigmenten,

. Schaumregulierungsmitteln wie Aluminiumstearat, hydrophobes Siliciumdioxid, Ethylen-bis-stearamid,

. natürlichem, gelöschten oder wasserhaltigem (hydroïque) Kalk,

. Gips,

. Wirkstoffen wie Pflanzenschutzmitteln,

. in Wasser löslichen basischen Verbindungen wie Natriummetasilicaten, Polysilicaten, Tone, Talk, Glimmer.

32. Vorläufersystem einer Bohrflüssigkeit in Form von Schaum nach Anspruch 31, dadurch gekennzeichnet, daß die Menge an festem(n) Füllstoff(en) höchstens 50 Gew.-% des genannten Vorläufersystems ausmacht, vorzugsweise höchstens 20 Gew.-% und noch mehr bevorzugt höchstens 15 Gew.-%.

33. Verfahren zur Herstellung einer Bohrflüssigkeit in Form von Schaum durch Verdünnen des wie in einem der Ansprüche 28 bis 32 definierten Vorläufersystems in Wasser und Rühren in der Weise, daß die am Schluß vorliegende Menge des oberflächenaktiven Mittels (TA) des Vorläufersystems in Wasser zwischen 0,05 Gew.-% und 25 Gew.-%, vorzugsweise zwischen 0,05 Gew.-% und 15 Gew.-%, und noch mehr bevorzugt zwischen 0,2 Gew.-% und 1,5 Gew.-% beträgt, bezogen auf das Wasser.

34. Verwendung der Bohrflüssigkeit in Form von Schaum, erhalten mit Hilfe des in irgendeinem der Ansprüche 28 bis 32 definierten Vorläufersystems oder mit Hilfe des in Anspruch 33 definierten Verfahrens, für das Ausschachten von Tunnels.

## Claims

1. A gel of an apolar medium characterised in that it contains:

- at least one apolar medium (MApo),
- at least one 'multicatenary' liquid surface active agent in acid form (ATMC) which is soluble or dispersible in said apolar medium,
- at least one neutralisation agent (AN) for said 'multicatenary' surface active agent present in an amount corresponding to 2 to 30 times and preferably 4 to 20 times the amount stoichiometrically necessary for neutralisation of said 'multicatenary' surface active agent in acid form (ATMC),

- from 0.2 to 5 and preferably from 0.3 to 3 molecules of water per molecule of 'multicatenary' liquid surface active agent in acid form (ATMC),

the water being introduced at least in part by way of said 'multicatenary' surface active agent in acid form (ATMC) and/or by way of said neutralisation agent (AN),

- optionally at least one emulsifying agent capable of emulsifying said gel in water or in an aqueous medium, and
- optionally at least one filler which is solid and soluble or insoluble in the apolar medium (MApo).

2. A gel according to claim 1 characterised in that the apolar medium (MApo) is selected from triglycerides of fatty acids which are saturated or unsaturated having at least 12 carbon atoms and preferably from 14 to 20 carbon atoms, aromatic petroleum cuts, terpene compounds, mixtures of dimethyl, dipropyl, diisobutyl and dibutyl succinate/adipate/glutarate diesters, aliphatic hydrocarbons containing at least 6 carbon atoms, aromatic solvents, chlorinated solvents and essential oils.

3. A gel according to claim 1 or claim 2 characterised in that the neutralisation agent (AN) for said 'multicatenary' surface active agent (ATMC) is liquid and non-soluble in said apolar suspension medium.

4. A gel according to claim 3 characterised in that the 'multicatenary' surface active agent (ATMC) is selected from surface active agents based on:

- phosphate diesters of alkoxylated alcohols, having from 2 to 20 and preferably from 4 to 10 oxyalkylene and preferably oxyethylene units, and for which the alcohols are selected from saturated or unsaturated, $C_6$-$C_{30}$ and preferably $C_6$-$C_{20}$ branched or straight-chain aliphatic alcohols, or
- phosphate diesters of ethoxylated alkylphenols, having from 2 to 20 and preferably from 4 to 10 oxyalkylene and preferably oxyethylene units, and for which the alkylphenols are selected from those in which the alkyl radical is $C_6$-$C_{30}$ and preferably $C_6$-$C_{20}$.

5. A gel according to claim 4 characterised in that the neutralisation agent (AN) is selected from primary, secondary or tertiary amines and alkanolamines.

6. A gel according to claim 5 characterised in that the neutralisation agent (AN) is selected from triethanolamine, aminomethylpropanol, cocoamine and butylamine.

7. A gel according to any one of claims 1 to 6 characterised in that the agent capable of emulsifying said gel in water or in an aqueous medium is selected from ethoxylated tristyrylphenols, sulphates or phosphates of ethoxylated and/or propoxylated tristyrylphenols, ethoxylated and/or propoxylated fatty alcohols or acids, ethoxylated/propoxylated block copolymers and sodium dodecylbenzenesulphonate.

8. A gel according to any one of claims 1 to 7 characterised in that the solid fillers are selected from silica, calcium carbonate, pigments, hydrocolloids capable of thickening the aqueous media, anti-foam agents, phytosanitary compounds, sodium metasilicates, clays, titanium oxide and zinc oxide.

9. A gel according to claim 8 characterised in that the hydrocolloids capable of thickening the aqueous media are selected from:

- polysaccharides obtained by bacterial fermentation,
- galactomannans,
- carrageenans,
- alginates,
- semisynthetic derivatives of cellulose,
- polyacrylates of alkali metals,
- polyvinyl alcohols,
- polyethylene glycols,
- polyvinylpyrrolidones,

alone or in association with each other.

10. A gel according to any one of the preceding claims characterised in that the amount of 'multicatenary' liquid surface active agent (ATMC) is from 0.5 to 10% and preferably from 1 to 6% by weight with respect to said gel.

11. A gel according to any one of the preceding claims characterised in that it is 'flexible' (or 'weak'), that the amount of 'multicatenary' surface active agent (ATMC) is from 1 to 3% by weight with respect to the weight of said gel, with a neutralisation agent (AN)/'multicatenary' surface active agent (ATMC) molar ratio of from 5 to 10 times the stoichiometric neutralisation ratio, and that the amount of water is from 0.01 to 0.1 % by weight with respect to the weight of said gel.

12. A gel according to any one of claims 1 to 10 characterised in that it is 'hard' (or 'strong'), that the amount of 'multicatenary' surface active agent (ATMC) is from 5 to 10% by weight with respect to the weight of said gel, with a neutralisation agent (AN)/'multicatenary' surface active agent (ATMC) molar ratio of from 5 to 10 times the stoichiometric neutralisation ratio, and that the amount of water is from 0.05 to 0.5% by weight with respect to the weight of said gel.

13. A gel (gel G1) according to any one of claims 1 to 9 characterised in that it comprises:

- 100-(a+b+c+d) parts by weight of at least one apolar medium (MApo),
- an amount (a) of from 0.5 to 6 and preferably from 1 to 4 parts by weight of at least 'multicatenary' liquid surface active agent in acid form (ATMC) which is soluble or dispersible in said apolar medium,
- an amount (b) of at least one neutralisation agent (AN) for said 'multicatenary' surface active agent corresponding to from 2 to 30 times and preferably from 4 to 20 times the amount stoichiometrically necessary for neutralisation of said 'multicatenary' surface active agent in acid form (ATMC),
- an amount (c) of water corresponding to from 0.2 to 5 and preferably from 0.3 to 3 molecules of water per molecule of 'multicatenary' liquid surface active agent in acid form (ATMC),
    the water being introduced at least in part by way of said 'multicatenary' surface active agent in acid form (ATMC) and/or by way of said neutralisation agent (AN), and
- an amount (d) of from 10 to 50 and preferably from 20 to 40 parts by weight of a filler formed by at least one hydrocolloid capable of thickening the aqueous media.

14. A gel (gel G1) according to claim 13 characterised in that the hydrocolloid capable of thickening the aqueous media is selected from xanthane gum, polysuccinoglycanes, rhamsan, wellan and gellan.

15. A gel (gel G1) according to claim 13 characterised in that the hydrocolloid capable of thickening the aqueous media is a mixture (M) containing:

- at least one hydrocolloid polysaccharide obtained by bacterial fermentation (PSB) which is stable in the presence of $Ca^{2+}$ ions at a pH-value of higher than 10 and having a pseudo-plasticity index of less than or equal to 0.5 at a concentration of 0.1% by weight in distilled water, and
- at least one hydrocolloid polymer of natural origin (PN) which is stable in the presence of cement and $Ca^{2+}$ ions at a pH-value of higher than 10 and having a pseudo-plasticity index of higher than or equal to 0.6 at a concentration of 0.3% by weight in distilled water,

    in accordance with a PN/PSB ratio by weight of 20/80 to 95/5, preferably from 50/50 to 90/10.

16. A gel (gel G1) according to claim 15 characterised in that the hydrocolloid polysaccharide obtained by bacterial fermentation (PSB) is a polysuccinoglycane, a rhamsan rubber or a wellan rubber.

17. A gel (gel G1) according to claim 15 or claim 16 characterised in that the hydrocolloid polymer of natural origin (PN) is a semisynthetic derivative of cellulose, or an alkoxylated derivative of guar rubber containing per molecule from 0.01 to 5 and preferably from 0.05 to 0.5 oxyalkylene units.

18. A gel (gel G1) according to claim 17 characterised in that the hydrocolloid polymer of natural origin (PN) is a hydroxymethylcellulose, hydroxyethylcellulose, hydroxyrnethylpropylcellulose, hydroxypropyl-cellulose, carboxymethylcellulose, or a hydroxyalkylguar containing per mole from 0.01 to 5 and preferably from 0.05 to 0.5 oxyethylene and/or oxypropylene units.

19. A gel according to one of claims 15 to 18 characterised in that the mixture (M) contains:

- a polysaccharide succinoglycane, and
- a hydroxyalkylguar containing from 0.01 to 5 and preferably from 0.05 to 0.5 oxyethylene and/or oxypropylene units,

  in a hydroxyalkylguar/succinoglycane ratio by weight of from 20/80 to 95/5 and preferably from 50/50 to 90/10.

**20.** A process for gelling an apolar medium (MApo) characterised by introducing into said apolar medium:

- at least one 'multicatenary' liquid surface active agent in acid form (ATMC) which is soluble or dispersible in said apolar medium,
- at least one neutralisation agent (AN) for said 'multicatenary' surface active agent present in an amount corresponding to 2 to 30 times and preferably 4 to 20 times the amount stoichiometrically necessary for neutralisation of said 'multicatenary' surface active agent in acid form (ATMC),
- from 0.2 to 5 and preferably from 0.3 to 3 molecules of water per molecule of 'multicatenary' liquid surface active agent in acid form (ATMC),
    the water being introduced at least in part by way of said 'multicatenary' surface active agent in acid form (ATMC) and/or by way of said neutralisation agent (AN),
- optionally at least one emulsifying agent capable of emulsifying the gel obtained in water or in an aqueous medium, and
- optionally at least one filler which is solid and soluble or insoluble in the apolar medium (MApo).

**21.** A process according to claim 20 characterised in that it comprises introducing with mechanical agitation the 'multicatenary' surface active agent (ATMC) in the apolar medium (MApo), then the optional emulsifying agent, the neutralisation agent (AN) and the optional fillers.

**22.** A process according to claim 20 or claim 21 characterised in that the different compounds used are selected from those occurring in the composition of the gel as set forth in any one of claims 2 to 9 and 14 to 19.

**23.** A process according to any one of claims 20 to 22 characterised in that the different compounds are used in accordance with amounts corresponding to the respective amounts of the constituents of the gel as set forth in any one of claims 13 to 16.

**24.** Use of the gel defined as set forth in any one of claims 13 to 19 for the preparation of a water-based drilling fluid.

**25.** Use according to the preceding claim for the preparation of a water-based drilling fluid for the production of moulded walls.

**26.** A process for the preparation of a water-based drilling fluid characterised by diluting in water the gel (gel G1) defined as set forth in one of claims 13 to 19 in such a way that the fluid comprises from 0.01 to 3 parts by weight of hydrocolloid capable of thickening the aqueous medium, introduced by way of the gel (gel G1), preferably as from 0.05 to 1.5 part by weight for 100 parts of water.

**27.** Use of the gel (gel G1) defined as set forth in one of claims 13 to 19 in association with a surface active agent (TA) as a precursor system for a drilling fluid in foam form.

**28.** A precursor system for a drilling fluid in foam form characterised in that it comprises the gel (gel G1) defined as set forth in one of claims 13 to 19 and a surface active agent (TA).

**29.** A precursor system for a drilling fluid in foam form according to claim 28 characterised in that the surface active agent (TA) is selected from:

- alkylsulphates of the formula $ROSO_3M$ in which R represents a $C_5$-$C_{24}$ and preferably $C_{10}$-$C_{18}$ alkyl or hydroxyalkyl radical, M representing a hydrogen atom or an alkali metal cation (sodium, potassium, lithium), substituted or unsubstituted ammonium (methyl, dimethyl, trimethyl, tetramethylammonium, dimethylpiperidinium) or a derivative of an alkanolamine (monoethanolamine, diethanolamine, triethanolamine, and monoisopropanolamine);
- betanes, alkylbetanes, alkyldimethylbetanes, alkylamidobetanes and alkyltrimethylsulphobetanes;
- mixtures of sodium alkylsulphosuccinates and alkylamidobetanes in which the alkyl radical is $C_4$-$C_{18}$;

- alkyl ether sulphates $RO(CH_2CHR_1O)_nSO_3M$ in which n is between 0.5 and 30, preferably between 0.5 and 10, and in which $R_1$ is a $C_4$-$C_{18}$ alkyl group, and having on average from 0.5 to 30 units and preferably from 0.5 to 10 units of oxyethylene and/or oxypropylene, M representing a hydrogen atom or an alkali metal cation (sodium, potassium, lithium), substituted or unsubstituted ammonium (methyl, dimethyl, trimethyl, tetramethylammonium, dimethylpiperidinium) or a derivative of an alkanolamine (monoethanolamine, diethanolamine, triethanolamine, and monoisopropanolamine);
- alpha olefin sulphonates;
- alkylpolyglycosides of the formula $R(OCH_2CH_2)_n(G)_m$, in which R is a saturated or unsaturated $C_8$-$C_{24}$ branched or straight-chain hydrocarbon radical, G is a reducing $C_5$-$C_6$ sugar such as galactose, glucose or fructose, n being between 0 and 20 and m being between 1 and 10, and
- alkylamphomonoacetates, alkylamphodiacetates such as sodium cocoamphoacetate.

30. A precursor system for a drilling fluid in foam form according to one of claims 28 and 29 characterised in that the content of surface active agent (TA) is between 1 and 2000% by weight with respect to the weight of the gel (gel G1).

31. A precursor system for a drilling fluid in foam form according to any one of claims 28 to 30 characterised in that it further comprises at least one solid filler selected from:

- natural or synthetic silica,
- calcium carbonate and magnesium carbonate,
- metallic oxides such as iron oxide,
- natural or synthetic pigments,
- foam-regulating agents such as aluminium stearate, hydrophobic silica, ethylene bis stearamide,
- natural, slaked and hydroic lime,
- gypsum,
- active materials such as phytosanitary compounds, and
- water-soluble basic compounds such as sodium metasilicates, polysilicates, clays, talcs and mica.

32. A precursor system for a drilling fluid in foam form according to claim 31 characterised in that the amount of solid filler or fillers represents at most 50% by weight of said precursor system, preferably at most 20% and even more preferably at most 15%.

33. A process for the preparation of a drilling fluid in foam form by dilution in water of the precursor system defined as set forth in one of claims 28 to 32 and agitation in such a way that the final amount in water of the surface active agent (TA) of the precursor system is between 0.05 and 25% by weight with respect to the water, preferably between 0.05 and 15% and even more preferably between 0.2 and 1.5%.

34. Use of the drilling fluid in foam form obtained by means of the precursor system defined as set forth in any one of claims 28 to 32 or by the process defined as set forth in claim 33 for the excavation of tunnels.

**Fig. 1**

**Fig. 2**